# EUROPEAN PATENT SPECIFICATION

(11) **EP 2 751 573 B1**
(45) Date of publication and mention of the grant of the patent: **08.11.2017**
(21) Application number: 12828908.9
(22) Date of filing: 04.09.2012
(51) Int. Cl.: G01N 33/68, G01N 30/72

(54) **SYSTEM AND METHOD FOR THE DETECTION OF OVALBUMIN**
SYSTEM UND VERFAHREN ZUM NACHWEIS VON OVALBUMIN
SYSTÈME ET PROCÉDÉ POUR LA DÉTECTION D'OVALBUMINE

(30) Priority: 02.09.2011 US 201161530612 P
(43) Date of publication of application: 09.07.2014
(60) Divisional application: 17193549.7
(73) Proprietor: DH Technologies Development Pte. Ltd., Singapore 739256 (SG)
(72) Inventor: LOCK, Stephen J., Pudsley West Yorkshire LS285EW (GB); PURKAYASTHA, Subhasish, Acton, MA 01720 (US); WILLIAMSON, Brian L., Ashland, MA 01721 (US); DONG, Keling, Westborough, MA 01581 (US)
(74) Representative: J A Kemp
(86) International application number: PCT/US2012/053689
(87) International publication number: WO 2013/033713

(56) References cited:
- WO-A1-2007/031080
- WO-A1-2010/119261
- US-A1- 2003 219 838
- US-A1- 2005 064 515
- US-B2- 7 112 784
- US-B2- 7 901 942
- HEICK J ET AL: "First screening method for the simultaneous detection of seven allergens by liquid chromatography mass spectrometry", JOURNAL OF CHROMATOGRAPHY, ELSEVIER SCIENCE PUBLISHERS B.V, NL, vol. 1218, no. 7, 15 December 2010 (2010-12-15), pages 938-943, XP028138512, ISSN: 0021-9673, DOI: 10.1016/J.CHROMA.2010.12.067 [retrieved on 2010-12-23]
- KEVIN J. SHEFCHECK ET AL: "Confirmation of Peanut Protein Using Peptide Markers in Dark Chocolate Using Liquid Chromatography-Tandem Mass Spectrometry (LC-MS/MS)", JOURNAL OF AGRICULTURAL AND FOOD CHEMISTRY, vol. 54, no. 21, 1 October 2006 (2006-10-01), pages 7953-7959, XP055182984, ISSN: 0021-8561, DOI: 10.1021/jf060714e
- JING-TAO WU ET AL: "Protein digest analysis by pressurized capillary electrochromatography using an ion trap storage/reflectron time-of- flight mass detector", ANALYTICAL CHEMISTRY, AMERICAN CHEMICAL SOCIETY, US, vol. 69, no. 15, 1 August 1997 (1997-08-01), pages 2908-2913, XP008161723, ISSN: 0003-2700, DOI: 10.1021/AC970183G

## Description

The invention relates generally to kits and methods for detecting and quantifying allergens in samples, such as food samples, using mass spectrometry.

Food allergy can elicit harmful immune responses, e.g., as dermatitis, asthma, gastrointestinal impairment, anaphylaxis shock, etc. Conventional methods for detecting and/or quantifying allergens in food samples generally employ immunoassays, which can suffer from a number of shortcomings. For example, one such method known as Enzyme Linked Immunosorbent Assay (ELISA) requires the use of expensive kits and is semi-quantitative at best. Additionally, conventional immunoassays are generally limited to a single allergen in each screen and frequently result in false positives due to a lack of specificity.

Accordingly, there is a need for improved kits and methods for detecting and quantifying allergens in food samples.

Shefcheck, K. J. et al. (2006) Confirmation of Peanut Protein Using Peptide Markers in Dark Chocolate Using Liquid Chromatography-Tandem Mass Spectrometry (LC-MS/MS), J Agric. Food Chem., 54, 7953-7959; Heick, J., et al (2011), First screening method for the simultaneous detection of seven allergens by liquid chromatography mass spectrometry, Journal of Chromatography A, 1218, 938-943; US 2003/0219838 A1; and US 2005/0064515 A1 disclose methods for detecting proteins in a sample using mass spectrometry.

The invention is defined in the claims. The present invention provides methods and kits for detecting and/or quantifying ovalbumin in a sample using mass spectrometry. As described below, these methods and kits can enable the detection and/or quantitation of ovalbumin in a sample by taking advantage of one or more amino acid sequences specific to ovalbumin. For example, allergen-specific tryptic peptides can be detected using Liquid Chromatography-Tandem Mass Spectrometry (LC-MS/MS). In many embodiments, selected Multiple Reaction Monitoring (MRM) transitions can be observed for each peptide to enable a reliable quantitation of the allergen in a food sample, for example.

A set of peptides specific to various allergens is disclosed. For example, each of the allergens ovalbumin, lysozyme, casein (isoform S1 and S2), lactoglobulin, glutens containing high and low molecular weight glutenins, wheat, rye, oats, barley, mustard, sesame and various nuts can be detected and/or quantified using an isolated peptide specific to that allergen. In some cases, these allergen terms are broad definitions which encompasses various types of peptides from different proteins and in some cases different genus and/or species of allergens. For example, the term mustard encompasses allergens obtained from species in both the genus Brassica and Genus Sinapis, both understood to be forms of mustard. By way of example, an isolated peptide specific to ovalbumin, an allergen found in chicken eggs, can have one or more of the amino acid sequence of SEQ ID NOS: 1-7. An isolated peptide specific to lysozyme, an allergen also found in chicken eggs, can have one or more of the amino acid sequence of SEQ ID NOS: 8-11. An isolated peptide specific to casein S1, a milk protein, can have one or more of the amino acid sequence of SEQ. ID NOS. 12-18. An isolated peptide specific to casein S2, another isoform of the milk protein casein, can have one or more of the amino acid sequence of SEQ. ID NOS. 19-23. Isolated peptides specific to lactoglobulin, another allergen found in milk, can have one or more of the amino acid sequences of SEQ. ID NOS. 24-29. An isolated peptide specific to barley proteins, can have one or more of the amino acid sequence of SEQ. ID NOS. 30-33. An isolated peptide specific to low molecular weight glutenin, can have one or more of the amino acid sequence of SEQ. ID NOS. 34-59. An isolated peptide specific to high molecular weight glutenin, can have one or more of the amino acid sequence of SEQ. ID NOS. 60-79. An isolated peptide specific to proteins of various species classified as mustard, can have one or more of the amino acid sequence of SEQ. ID NOS. 80-112. An isolated peptide specific to oat proteins, can have one or more of the amino acid sequence of SEQ. ID NOS. 113-116. An isolated peptide specific to rye proteins, can have one or more of the amino acid sequence of SEQ. ID NOS. 117-123. An isolated peptide specific to sesame proteins, can have one or more of the amino acid sequence of SEQ. ID NOS. 124-132. An isolated peptide specific to wheat proteins, can have one or more of the amino acid sequence of SEQ. ID NOS. 133-138. An isolated peptide specific to brazil nut proteins, can have one or more of the amino acid sequence of SEQ. ID NOS. 139-155. An isolated peptide specific to hazelnut proteins, can have one or more of the amino acid sequence of SEQ. ID NOS. 156-172. An isolated peptide specific to macadamia nut proteins, can have one or more of the amino acid sequence of SEQ. ID NOS. 173-181. An isolated peptide specific to peanut proteins, can have one or more of the amino acid sequence of SEQ. ID NOS. 182-187. An isolated peptide specific to pistachio nut proteins, can have one or more of the amino acid sequence of SEQ. ID NOS. 188-203 and finally an isolated peptide specific to walnut proteins, can have one or more of the amino acid sequence of SEQ. ID NOS 204-212.

Each of the above-described peptides can be detected and/or quantified using specific MRM transitions associated with each of the allergen-specific peptides. Optimized MRM transitions for each of the peptides, according to various embodiments, is discussed below in Tables 1-19.

In one aspect, a method of screening a sample for ovalbumin is provided. The method includes obtaining a sample and digesting the sample with at least one proteolytic enzyme (e.g., trypsin) to fragment ovalbumin present in the sample, if any, into a plurality of peptides. The method additionally includes quantifying ovalbumin by determining an amount of at least one of the peptides using liquid chromatography tandem mass spectrometry (LC-MS/MS).

In some embodiments, ovalbumin is detected and/or quantified by detecting, via LC-MS/MS, multiple peptides such as those discussed above, where for each peptide fragment multiple MRM transitions, such as those discussed above, are monitored. It has been discovered that accurate identification and/or quantification of allergens (e.g., egg proteins such as ovalbumin, lysozyme or milk proteins such as casein and lactoglobulin, high and low glutenins, proteins obtained from wheat, rye, oats, barley, mustard, sesame and various nuts) can be achieved by monitoring and quantifying at least one and preferably several (e.g., all) of a set of specific peptides of the allergen (such as those disclosed herein) and specific MRM transitions for each peptide (such as those disclosed herein) using LC-MS/MS.

In one embodiment, at least one selected MRM transition can be monitored for each of the plurality of peptides. For example, two, three, or any other number of MRM transitions can be monitored for each of said peptides. In one embodiment, two or more allergens can be detected and/or quantified, for example, using the same run of a tandem mass spectrometer. In another embodiment, the amount of two or more peptides is determined to quantify the allergen of interest.

In some embodiments, the method can comprise adding a known amount of ovalbumin prior to digesting the sample.

Also disclosed herein is a method of detecting ovalbumin in a sample. The method includes adding a proteolytic enzyme (e.g., trypsin) to the sample to lyse at least a portion of the ovalbumin present in the sample, if any, into a plurality of peptides having at least one peptide having an amino acid sequence of SEQ ID NOS: 1,3,5 and 6. Liquid chromatography tandem mass spectrometry (LC-MS/MS) is then utilized to determine whether the at least one peptide is in the sample.

Selected MRM transitions can be monitored to detect and/or quantify selected peptides of the ovalbumin. For example, one selected precursor-product ion pair transitions can be monitored. Optimized MRM transitions for each of the peptides corresponding to SEQ ID NOS: 1-7 are provided below in Table 1. Any number of the optimized transitions for peptides of ovalbumin can be monitored via LC-MS/MS. For example, one, two, three, or optionally, all of the optimized MRM transitions can be monitored.

Also disclosed herein is a kit for use in the mass spectrometric testing of a sample for the allergen ovalbumin. The kit includes at least one proteolytic enzyme (e.g., trypsin) for fragmenting ovalbumin present in the sample, if any, into a plurality of peptides. The kit also includes at least one peptide having amino acid sequence of SEQ ID NO: 1 or 3.

In one aspect, the reagent for quantifying at least one of the plurality of peptides is an isotopically-enriched peptide having an amino acid sequence of SEQ ID NO: 1 or 3. In one embodiment, the isotopically-enriched peptide can include, for example, at least one of C13 or N15. In another aspect, the reagent for quantifying at least one said plurality of peptides can include a selected concentration of at least one said plurality of peptides having an amino acid sequence of SEQ ID NO: 1 or 3.

The kit can additionally include various agents used in preparing a sample to be analyzed using a mass spectrometer, running the prepared sample through a LC column, or performing mass spectrometry. By way of non-limiting example, the kit can include one of ovalbumin, lysozyme, casein, lactoglobulin, high or low glutenins, proteins from wheat, rye, oats, barley, mustard, sesame and various nuts including macadamia, pistachio, brazil, walnuts, peanuts and hazelnuts to be used as a standard, for example. The kit can also include an alkylating agent (e.g., Methyl methanethiosulfonate (MMTS), iodoacetamide) and/or a reducing agent.

In one aspect, the kit can include instructions for quantifying ovalbumin using a mass spectrometer. By way of example, the kit can include information pertaining to transition pairs to be used as settings in a mass spectrometer or the fragment ions that are expected.

According to various aspects, software is provided which can control the processes and/or perform the calculations described herein. For example, the software can provide instructions to a mass spectrometer to monitor one or more specific precursor-product ion pair transitions or fragment ions.

Those skilled in the art will understand that the methods and kits described herein are non-limiting exemplary embodiments and that the scope of the applicants' disclosure is defined solely by the claims. While the applicant's teachings are described in conjunction with various embodiments, it is not intended that the applicant's teachings be limited to such embodiments. On the contrary, the applicant's teachings encompass various alternatives, modifications, and equivalents that fall within the scope of the claims. The features illustrated or described in connection with one exemplary embodiment may be combined with the features of other embodiments. Such modifications and variations are intended to be included within the scope of the applicants' disclosure.

According to various embodiments, methods are provided for screening a sample for the presence or quantity of ovalbumin by detecting one or more amino acid sequences specific to ovalbumin. For example, ovalbumin-specific peptides can be detected using Liquid Chromatography-Tandem Mass Spectrometry (LC-MS/MS). Selected MRM transitions can be observed for each peptide to enable quantitation of ovalbumin in the sample.

The methods can utilize a variety of mass spectrometry techniques known in the art. For example, the mass spectrometry technique can be a tandem mass spectrometry (MS/MS) technique and/or a liquid chromatography tandem mass spectrometry (LC-MS/MS) technique. In some embodiments, the technique comprises an LC-MS/MS technique and the use of a triple quadrupole instrument and Multiple Reaction Monitoring (MRM).

The method comprises detecting and/or quantifying ovalbumin in the sample by detecting at least one isolated peptide specific to ovalbumin, namely, one or more of the peptides having an amino acid sequence of SEQ ID NOS: 1,3,5 and 6 identified herein. For example, LC-MS/MS can be used to determine the presence and/or quantity of ovalbumin-specific peptides in the sample. In one exemplary embodiment, a triple quadrupole mass spectrometer can be used to monitor selected Multiple Reaction Monitoring (MRM) transitions. In some embodiments, the ovalbumin-specific peptide can have the amino acid sequence of SEQ ID NO: 1 identified herein, and the method can include monitoring at least one precursor-product ion pair transition having an m/z value of about 391/504,391/667, or 391/433, wherein the term "about" as used herein means within a range of +/- one (1) atomic mass unit. In unclaimed examples, the ovalbumin-specific peptide can have the amino acid sequence of SEQ ID NO: 2 identified herein, and the method can include monitoring at least one precursor-product ion pair transition having an m/z value of about 762/1036, 762/487, 762/1150. In other embodiments, the ovalbumin-specific peptide can have the amino acid sequence of SEQ ID NO: 3 identified herein, and the method can include monitoring at least one precursor-product ion pair transition having an m/z value of about 605/419,605/621, or 605,749. In unclaimed examples, the ovalbumin-specific peptide can have the amino acid sequence of SEQ ID NO: 4, and the method can include monitoring at least one precursor-product ion pair transition having an m/z value of about 673/1096,673/1024,673/1209,449/526,449/608, or 449/639. In other embodiments, the ovalbumin-specific peptide can have the amino acid sequence of SEQ ID NO: 5 identified herein, and the method can include monitoring at least one precursor-product ion pair transition having an m/z value of about 791/1052, 791/951, or 791/1239. In other embodiments, the ovalbumin-specific peptide can have the amino acid sequence of SEQ ID NO: 6 identified herein, and the method can include monitoring at least one precursor-product ion pair transition having an m/z value of about 844/666,844/1332, or 844/1121. In unclaimed examples, the ovalbumin-specific peptide can have the amino acid sequence of SEQ ID NO: 7, and the method can include monitoring at least one precursor-product ion pair transition having an m/z value of about 930/1017, 930/1118, 930/1303, or 930/888.

The method includes monitoring multiple precursor-product ion pair transitions associated with each ovalbumin-specific peptide. By way of example, the ovalbumin-specific peptide having the amino acid sequence of SEQ ID NO: 1 can be identified by monitoring any two of the precursor-product ion pair transitions having an m/z value of about 391/504,391/667, or 391/433. In one embodiment, all three of the identified precursor-product ion pairs can be monitored.

The method for detecting or quantifying ovalbumin in the sample includes detecting multiple ovalbumin-specific peptides. By way of non-limiting example, ovalbumin can be quantified by using any combination, or all, of the peptides having the amino acid sequences of SEQ ID NOS: 1-7 identified herein. As discussed above, for each peptide, one or more of the precursor-product ion pair transitions can be monitored.

Table 1 below shows sequences SEQ ID NOS: 1-7 of the peptides determined, according to applicants' teachings, to be specific to ovalbumin, along with their optimal MRM Q1, Q3 transitions. These observed peptides and transitions can be used to enable a reliable quantitation of the ovalbumin present in the sample. Additional MRM transitions specific to ovalbumin are shown in Appendix A, B, and D.

In the following tables the Fragment Ion has a reference to the charge as well as the conventional fragmentation nomenclature. For example, the label of 2y4 would indicate a fragment that is doubly charged and is the y4 fragment (four units from the C-terminus). Likewise a label of 2b4 would indicate a fragment that is doubly charged and is the b4 fragment (four units from the N-terminus). These nomenclature rules are known to those skilled in the art.

**TABLE 1**

| Ovalbumin (Chicken) | Q1 | Q3 | Fragment Ion |
|---|---|---|---|
| LYAEER (SEQ ID NO: 1) | 391.1 | 504.3 | 2y4 |
| | 391.1 | 667.4 | 2y5 |
| doubly-charged | 391.1 | 433.2 | 2y3 |
| YPILPEYLQCVK (SEQ ID NO: 2) | 762.3 | 1036.7 | 2y8 |
| | 762.3 | 487.4 | 2b4 |
| doubly-charged | 762.3 | 1149.9 | 2y9 |
| DEDTQAMPFR (SEQ ID NO: 3) | 605.7 | 419.3 | 2y3 |
| | 605.7 | 621.6 | 2y5 |
| doubly-charged | 605.7 | 749.5 | 2y6 |
| HIATNAVLFFGR (SEQ ID NO: 4) | 673.5 | 1095.9 | 2y10 |
| | 673.5 | 1024.7 | 2y9 |
| doubly-charged | 673.5 | 1209 | 2y11 |
| HIATNAVLFFGR (SEQ ID NO: 4) | 449.2 | 526.5 | 2y10 |
| | 449.2 | 608.5 | 2y9 |
| triply-charged | 449.2 | 639.5 | 2y11 |
| LTEWTSSNVMEER (SEQ ID NO: 5) | 791.7 | 1052.7 | 2y10 |
| | 791.7 | 951.6 | 2y9 |
| doubly-charged | 791.7 | 1238.8 | 2y11 |
| GGLEPINFQTAADQAR (SEQ ID NO: 6) | 844.6 | 666.7 | 2y10 |
| | 844.6 | 1331.9 | 2y9 |
| doubly-charged | 844.6 | 1121.6 | 2y11 |
| ELINSWVESQTNGIIR (SEQ ID NO: 7) | 930.5 | 1017.5 | 2y12d |
| doubly-charged | 930.5 | 1117.8 | 2y11 |
| | 930.5 | 1302.9 | 2y10 |
| | 930 | 888.5 | |

According to unclaimed examples, the method can comprise detecting and/or quantifying lysozyme in the sample by detecting at least one isolated peptide specific to lysozyme, for example, one or more of the peptides having an amino acid sequence of SEQ ID NOS: 8-11 identified herein. For example, a triple quadrupole mass spectrometer can be used to monitor selected Multiple Reaction Monitoring (MRM) transitions to determine the presence and/or quantity of lysozyme-specific peptides in the sample. The lysozyme-specific peptide can have the amino acid sequence of SEQ ID NO: 8 identified herein, and the method can include monitoring at least one precursor-product ion pair transition having an m/z value of about 438/737,438/452, or 438/680. The lysozyme-specific peptide can have the amino acid sequence of SEQ ID NO: 9 identified herein, and the method can include monitoring at least one precursor-product ion pair transition having an m/z value of about 497/621,497/847, or 497/807. The lysozyme-specific peptide can have the amino acid sequence of SEQ ID NO: 10 identified herein, and the method can include monitoring at least one precursor-product ion pair transition having an m/z value of about 715/804, 715/951, or 715/1065. The lysozyme-specific peptide can have the amino acid sequence of SEQ ID NO: 11, and the method can include monitoring at least one precursor-product ion pair transition having an m/z value of about 878/901, 878/1064, or 878/1178.

In one unclaimed example, the method can include monitoring multiple precursor-product ion pair transitions associated with each lysozyme-specific peptide. By way of example, the lysozyme-specific peptide having the amino acid sequence of SEQ ID NO: 8 can be identified by monitoring any two of the precursor-product ion pair transitions having an m/z value of about 438/737, 438/452, or 438/680. All three of the identified precursor-product ion pairs can be monitored.

In one unclaimed example, the method for detecting or quantifying lysozyme in the sample can include detecting multiple lysozyme-specific peptides. By way of non-limiting example, lysozyme can be quantified by using any combination, or all, of the peptides having the amino acid sequences of SEQ ID NOS: 8-11 identified herein. As discussed above, for each peptide, one or more of the precursor-product ion pair transitions can be monitored.

Table 2 below shows sequences SEQ ID NOS: 8-11 of the peptides determined, according to the present teachings, to be specific to lysozyme, along with their optimal MRM Q1, Q3 transitions. According to various unclaimed examples, these observed peptides and transitions can be used to enable a reliable quantitation of the lysozyme present in the sample. Additional MRM transitions specific to lysozyme are shown in Appendix A, B, and D.

**TABLE 2**

| Lysozyme (Chicken) | Q1 | Q3 | Fragment Ion |
|---|---|---|---|
| HGLDNYR (SEQ ID NO: 8) | 438 | 737.5 | 2y6 |
| | 438 | 452.3 | 2y3 |
| doubly-charged | 438 | 680.3 | 2y5 |
| WWCNDGR (SEQ ID NO: 9) | 497.6 | 621.4 | 2y5 |
| | 497.6 | 847.5 | 2y6 |
| doubly-charged | 497.6 | 807.5 | |
| FESNFNTQATNR (SEQ ID NO: 10) | 715 | 804.7 | 2y7 |
| | 715 | 951.6 | 2y8 |
| doubly-charged | 715 | 1065.7 | 2y9 |
| NTDGSTDYGILQINSR SEQ ID NO: 11) | 878 | 901 | 2y8 |
| | 878 | 1064.3 | 2y9 |
| doubly-charged | 878 | 1178.7 | 2y10 |

According to some unclaimed examples, the method can comprise detecting and/or quantifying the casein in the sample by detecting at least one isolated peptide specific to casein, for example, one or more of the peptides having an amino acid sequence of SEQ ID NOS: 12-23 identified herein. For example, the method can comprise detecting or quantifying the S1 isoform of casein in the sample by detecting an isolated peptide specific to casein S1, e.g., one or more of the peptides having am amino acid sequence of SEQ ID NOS: 12-18 identified herein. By way of example, a triple quadrupole mass spectrometer can be used to monitor selected Multiple Reaction Monitoring (MRM) transitions to determine the presence and/or quantity of casein S1-specific peptides in the sample. The casein S1-specific peptide can have the amino acid sequence of SEQ ID NO: 12 identified herein, and the method can include monitoring at least one precursor-product ion pair transition having an m/z value of about 693/921,693/992, or 693/1091. The casein S1-specific peptide can have the amino acid sequence of SEQ ID NO: 13 identified herein, and the method can include monitoring at least one precursor-product ion pair transition having an m/z value of about 584/763, 584/706, 584/650, or 438/600. The casein S1-specific peptide can have the amino acid sequence of SEQ ID NO: 14 identified herein, and the method can include monitoring at least one precursor-product ion pair transition having an m/z value of about 416/488, 416/587, or 416/702. The casein S1-specific peptide can have the amino acid sequence of SEQ ID NO: 15, and the method can include monitoring at least one precursor-product ion pair transition having an m/z value of about 634/992, 634/771, or 634/934. The casein S1-specific peptide can have the amino acid sequence of SEQ ID NO: 16 identified herein, and the method can include monitoring at least one precursor-product ion pair transition having an m/z value of about 880/1325, 880/1495, 880/1438, 880/436, 587/758, 587/871, or 587/790. The casein S1-specific peptide can have the amino acid sequence of SEQ ID NO: 17 identified herein, and the method can include monitoring at least one precursor-product ion pair transition having an m/z value of about 345/590,345/476, or 345/573. The casein S1-specific peptide can have the amino acid sequence of SEQ ID NO: 18, and the method can include monitoring at least one precursor-product ion pair transition having an m/z value of about 416/488, 416/587, or 416/702.

In one unclaimed example, the method can include monitoring multiple precursor-product ion pair transitions associated with each casein S1-specific peptide. By way of example, the casein S1-specific peptide having the amino acid sequence of SEQ ID NO: 12 can be identified by monitoring any two of the precursor-product ion pair transitions having an m/z value of about 693/921, 693/992, or 693/1091. All three of the identified precursor-product ion pairs can be monitored.

In one unclaimed example, the method for detecting or quantifying the S1 isoform of casein in the sample can include detecting multiple casein S1-specific peptides. By way of non-limiting example, casein S1 can be quantified by using any combination, or all, of the peptides having the amino acid sequences of SEQ ID NOS: 12-18 identified herein. As discussed above, for each peptide, one or more of the precursor-product ion pair transitions can be monitored.

Table 3 below shows sequences SEQ ID NOS: 12-18 of the peptides determined, according to the applicants' teachings, to be specific to the S1 isoform of casein, along with their optimal MRM Q1, Q3 transitions. According to various unclaimed examples, these observed peptides and transitions can be used to enable a reliable quantitation of the S1 isoform of casein present in the sample. Additional MRM transitions specific to the S1 isoform of casein are shown in Appendix A, B, and D.

**TABLE 3**

| **Casein S1 (Bovine)** | **Q1** | **Q3** | **Fragment Ion** |
|---|---|---|---|
| **FFVAPFPEVFGK** | **693.3** | **921.6** | **2y8** |
| **(SEQ ID NO: 12)** | **693.3** | **991.9** | **2y9** |
| | | | |
| **doubly-charged** | **693.3** | **1091.5** | **2y10** |
| **LLILTCLVAVALARPK** | **584.4** | **763.5** | **3y14d** |
| **(SEQ ID NO: 13)** | **584.4** | **706.3** | **3y13d** |
| | | | |
| **triply-charged** | **584.4** | **650.4** | **3y12d** |
| **LLILTCLVAVALARPK** | | | |
| **(SEQ ID NO: 13)** | **438** | **599.9** | **4y11d** |
| | | | |
| **quadrupally-charged** | | | |
| **EDVPSER** | **416.4** | **488.3** | **2y4** |
| **(SEQ ID NO: 14)** | **416.4** | **587.4** | **2y5** |
| | | | **2y6** |
| **doubly-charged** | **416.4** | **702.7** | |
| **YLGYLEQLLR** | **634.5** | **991.8** | **2y8** |
| **(SEQ ID NO: 15)** | **634.5** | **771.7** | **2y6** |
| | | | **2y7** |
| **doubly-charged** | **634.5** | **934.9** | |
| **HQGLPQEVLNENLLR** | **880.6** | **1325** | **2y11** |
| **(SEQ ID NO: 16)** | **880.6** | **1495** | **2y13** |
| | | | |
| | **880.6** | **1438** | **2y12** |
| **doubly-charged** | | | |
| | **880.5** | **436.2** | |
| **HQGLPQEVLNENLLR** | **587.3** | **758.4** | |
| **(SEQ ID NO: 16)** | | | |
| | **587.3** | **871.5** | |
| **triply-charged** | | | |
| | **587.3** | **790.4** | |
| **VNELSK** | **345.3** | **590.5** | **2y5** |
| **(SEQ ID NO: 17)** | **345.3** | **476.3** | **2y4** |
| | | | **2y5w** |
| **doubly-charged** | **345.3** | **573.3** | |
| **EDVPSER** | **416.4** | **488.3** | **2y4** |
| **(SEQ ID NO: 18)** | **416.4** | **587.4** | **2y5** |
| | | | **2y6** |
| **doubly-charged** | **416.4** | **702.7** | |

According to some unclaimed examples, the method can comprise detecting and/or quantifying the S2 isoform of casein in the sample by detecting at least one isolated peptide specific to casein S2, e.g., one or more of the peptides having an amino acid sequence of SEQ ID NOS: 19-23 identified herein. By way of example, a triple quadrupole mass spectrometer can be used to monitor selected Multiple Reaction Monitoring (MRM) transitions to determine the presence and/or quantity of casein 31-specific peptides in the sample. The casein S2-specific peptide can have the amino acid sequence of SEQ ID NO: 19 identified herein, and the method can include monitoring at least one precursor-product ion pair transition having an m/z value of about 373/534 or 373/437. The casein S2-specific peptide can have the amino acid sequence of SEQ ID NO: 20 identified herein, and the method can include monitoring at least one precursor-product ion pair transition having an m/z value of about 598/912, 598/701, 598/814, 598/436. The casein S2-specific peptide can have the amino acid sequence of SEQ ID NO: 21 identified herein, and the method can include monitoring at least one precursor-product ion pair transition having an m/z value of about 438/629,438/558, or 438/445. The casein S2-specific peptide can have the amino acid sequence of SEQ ID NO: 22, and the method can include monitoring at least one precursor-product ion pair transition having an m/z value of about 490/648, 490/761, 490/833. The casein S2-specific peptide can have the amino acid sequence of SEQ ID NO: 23 identified herein, and the method can include monitoring at least one precursor-product ion pair transition having an m/z value of about 694/1187,694/811, or 694/940.

In one unclaimed example, the method can include monitoring multiple precursor-product ion pair transitions associated with each casein S2-specific peptide. By way of example, the casein S2-specific peptide having the amino acid sequence of SEQ ID NO: 19 can be identified by monitoring both of the precursor-product ion pair transitions having an m/z value of about 373/534 or 373/437. With reference to the casein S2-specific peptide having the amino acid sequence of SEQ ID NO: 20, any two, three, or all of the identified precursor-product ion pairs having an m/z value of about 598/912, 598/701, 598/814, or 598/436 can be monitored.

In one unclaimed example, the method for detecting or quantifying the S2 isoform of casein in the sample can include detecting multiple casein S2-specific peptides. By way of non-limiting example, casein S2 can be quantified by using any combination, or all, of the peptides having the amino acid sequences of SEQ ID NOS: 19-23 identified herein. As discussed above, for each peptide, one or more of the precursor-product ion pair transitions can be monitored.

Table 4 below shows sequences SEQ ID NOS: 19-23 of the peptides determined, according to the applicants' teachings, to be specific to the S2 isoform of casein, along with their optimal MRM Q1, Q3 transitions. According to various unclaimed examples, these observed peptides and transitions can be used to enable a reliable quantitation of the S2 isoform of casein present in the sample.

**TABLE 4**

| **Casein S2 (Bovine)** | **Q1** | **Q3** | **Fragment Ion** |
|---|---|---|---|
| **VIPYVR** | **373.7** | **534.4** | **2y4** |
| **(SEQ ID NO: 19)** | | | **2y3** |
| | **373.7** | **437.3** | |
| **doubly-charged** | | | |
| | **598.5** | **911.8** | **2y8** |
| **NAVPITPTLNR** | **598.5** | **701.6** | **2y6** |
| **(SEQ ID NO: 20)** | | | **2y7** |
| | **598.5** | **814.7** | |
| **doubly-charged** | | | |
| | **598.3** | **436.2** | |
| **NMAINPSK** | **438** | **629.5** | **2y6** |
| **(SEQ ID NO: 21)** | | | **2y5** |
| | **438** | **558.4** | **2y4** |
| **doubly-charged** | **438** | **445.4** | |
| **FALPQYLK** | **490.3** | **648.5** | **2y5** |
| **(SEQ ID NO: 22)** | **490.3** | **761.7** | **2y6** |
| | | | **2y7** |
| **doubly-charged** | | | |
| | **490.3** | **832.8** | |
| **TVDMESTEVFTK** | **694** | **1186.9** | **2y10** |
| **(SEQ ID NO: 23)** | **694** | **811.6** | **2y7** |
| | | | |
| **doubly-charged** | **694** | **940.7** | **2y8** |

According to some unclaimed examples, the method can comprise detecting and/or quantifying lactoglobulin in the sample by detecting at least one isolated peptide specific to lactoglobulin, for example, one or more of the peptides having am amino acid sequence of SEQ ID NOS: 24-29 identified herein. For example, a triple quadrupole mass spectrometer can be used to monitor selected Multiple Reaction Monitoring (MRM) transitions to determine the presence and/or quantity of lysozyme-specific peptides in the sample. The lactoglobulin-specific peptide can have the amino acid sequence of SEQ ID NO: 24 identified herein, and the method can include monitoring at least one precursor-product ion pair transition having an m/z value of about 533/853,533/754, or 533/641. The lactoglobulin-specific peptide can have the amino acid sequence of SEQ ID NO: 25 identified herein, and the method can include monitoring at least one precursor-product ion pair transition having an m/z value of about 458/803,458/688, or 458/504. The lactoglobulin-specific peptide can have the amino acid sequence of SEQ ID NO: 26 identified herein, and the method can include monitoring at least one precursor-product ion pair transition having an m/z value of about 1158/1453, 1158/1581, 1158/1255, 772/1026, 772/977, or 772/912. The lactoglobulin-specific peptide can have the amino acid sequence of SEQ ID NO: 27, and the method can include monitoring at least one precursor-product ion pair transition having an m/z value of about 623/573,623/918, 623/819,623/1047. The lactoglobulin-specific peptide can have the amino acid sequence of SEQ ID NO: 28 identified herein, and the method can include monitoring at least one precursor-product ion pair transition having an m/z value of about 561/806,561/935, or 561/692. The lactoglobulin-specific peptide can have the amino acid sequence of SEQ ID NO: 29, and the method can include monitoring at least one precursor-product ion pair transition having an m/z value of about 419/653,419/556, or 419/425.

In one unclaimed example, the method can include monitoring multiple precursor-product ion pair transitions associated with each lactoglobulin-specific peptide. By way of example, the lactoglobulin-specific peptide having the amino acid sequence of SEQ ID NO: 24 can be identified by monitoring any two of the precursor-product ion pair transitions having an m/z value of about 533/853,533/754, or 533/641. In one embodiment, all three of the identified precursor-product ion pairs can be monitored.

In one unclaimed example, the method for detecting and/or quantifying lactoglobulin in the sample can include detecting multiple lactoglobulin-specific peptides. By way of non-limiting example, lactoglobulin can be quantified by using any combination, or even all, of the peptides having the amino acid sequences of SEQ ID NOS: 24-29 identified herein. As discussed above, for each peptide, one or more of the precursor-product ion pair transitions can be monitored.

Table 5 below shows sequences SEQ ID NOS: 24-29 of the peptides determined, according to the applicants' teachings, to be specific to lactoglobulin, along with their optimal MRM Q1, Q3 transitions. According to various unclaimed examples, these observed peptides and transitions can be used to enable a reliable quantitation of the lactoglobulin present in the sample. Additional MRM transitions specific to lactoglobulin are shown in Appendix A, B, and D.

**TABLE 5**

| **Lactoglobulin (Bovine)** | **Q1** | **Q3** | **Fragment Ion** |
|---|---|---|---|
| **VLVLDTDYK** | **533.3** | **853.6** | **2y7** |
| **(SEQ ID NO: 24)** | **533.3** | **754.6** | **2y6** |
| | | | **2y5** |
| **doubly-charged** | **533.3** | **641.4** | |
| **IDALNENK** | **458.7** | **803.6** | **2y7** |
| **(SEQ ID NO: 25)** | **458.7** | **688.5** | **2y6** |
| | | | **2y4** |
| **doubly-charged** | **458.7** | **504.2** | |
| **VYVEELKPTPEGDLEILLQK** | **1158** | **1453.5** | **2y13** |
| **(SEQ ID NO: 26)** | **1158** | **1581.5** | **2y14** |
| | | | **2y11** |
| **doubly-charged** | **1158** | **1254.9** | |
| **VYVEELKPTPEGDLEILLQK** | **772.3** | **1026.5** | **3y18d** |
| **(SEQ ID NO: 26)** | **772.3** | **977** | **3y17d** |
| | | | **3y16d** |
| **triply-charged** | **772.3** | **912.5** | |
| **TPEVDDEALEK** | **623.3** | **572.9** | **2y10d** |
| **(SEQ ID NO: 27)** | | | |
| | **623.3** | **918.5** | **2y8** |
| **doubly-charged** | | | |
| | **623.3** | **819.5** | **2y7** |
| | | | |
| | **623.3** | **1047.5** | |
| **WENGECAQK** | **561.3** | **806.4** | **2y7** |
| **(SEQ ID NO: 28)** | **561.3** | **935.4** | **2y8** |
| | | | **2y6** |
| **doubly-charged** | **561.3** | **692.6** | |
| **ALPMHIR** | **419.2** | **653.3** | **2y5** |
| **(SEQ ID NO: 29)** | **419.2** | **555.9** | **2y4** |
| | | | **2y3** |
| **doubly-charged** | **419.2** | **424.9** | |

According to some unclaimed examples, the method can comprise detecting and/or quantifying barley (Hordeum vulgare) in the sample by detecting at least one isolated peptide specific to a barley, such as example the B1-hordein or B3-hordein which can have, for example, one or more of the peptides having an amino acid sequence of SEQ ID NOS: 30-33 identified herein. For example, a triple quadrupole mass spectrometer can be used to monitor selected Multiple Reaction Monitoring (MRM) transitions to determine the presence and/or quantity of barley-specific peptides in the sample. The barley-specific peptide can have the amino acid sequence of SEQ ID NO: 30 identified herein, and the method can include monitoring at least one precursor-product ion pair transition having an m/z value of about 835/948, 835/1097, or 835/1228. The barley-specific peptide can have the amino acid sequence of SEQ ID NO: 31 identified herein, and the method can include monitoring at least one precursor-product ion pair transition having an m/z value of about 336/515,336/554, or 336/497. The barley-specific peptide can have the amino acid sequence of SEQ ID NO: 32 identified herein, and the method can include monitoring at least one precursor-product ion pair transition having an m/z value of about 820/1097,820/548, or 820/713. The barley-specific peptide can have the amino acid sequence of SEQ ID NO: 33, and the method can include monitoring at least one precursor-product ion pair transition having an m/z value of about 499/785,499/575, or 499/393.

In one unclaimed example, the method can include monitoring multiple precursor-product ion pair transitions associated with each barley-specific peptide. By way of example, the barley-specific peptide having the amino acid sequence of SEQ ID NO: 30 can be identified by monitoring any two of the precursor-product ion pair transitions having an m/z value of about 835/948,835/1097, or 835/1228. All three of the identified precursor-product ion pairs can be monitored. Likewise, the amino acid sequence of SEQ ID NO: 31-33 can be used in a similar fashion.

In one unclaimed example, the method for detecting or quantifying barley in the sample can include detecting multiple barley-specific peptides. By way of non-limiting example, barley can be quantified by using any combination, or all, of the peptides having the amino acid sequences of SEQ ID NOS: 30-33 identified herein. As discussed above, for each peptide, one or more of the precursor-product ion pair transitions can be monitored.

Table 6 below shows sequences SEQ ID NOS: 30-33 of the peptides determined, according to the present teachings, to be specific to barley, along with their optimal MRM Q1, Q3 transitions. According to various unclaimed examples, these observed peptides and transitions can be used to enable a reliable quantitation of the barley proteins present in the sample.

**TABLE 6**

| **Barley Proteins** | **Q1** | **Q3** | **Fragment Ion** |
|---|---|---|---|
| **TLPMMCSVNVPLYR** | **835.4** | **947.5** | **2/y8** |
| **(SEQ ID NO: 30)** | **835.4** | **1096.5** | **2/y9** |
| | | | **2/y10** |
| **doubly-charged** | **835.4** | **1227.6** | |
| **GVGPSVGV** | **336.2** | **515.3** | **2/y6** |
| **(SEQ ID NO: 31)** | **336.2** | **554.3** | **2b7** |
| | | | **2b6** |
| **doubly-charged** | **336.2** | **497.3** | |
| **TLPTMCSVNVPLYR** | **820.4** | **1096.5** | **2/y8** |
| **(SEQ ID NO: 32)** | **820.4** | **548.3** | **2/y9(2)** |
| | | | |
| **doubly-charged** | **820.4** | **713.4** | **2/y12(2)** |
| **NTDGSTDYGILQINSR** | **499.3** | **785.5** | **2/y7** |
| **(SEQ ID NO: 33)** | | | **2/y5** |
| | **499.3** | **575.3** | **2/y7(2)** |
| **doubly-charged** | | | |
| | **499.3** | **393.3** | |

It is understood by those skilled in the art that in some cases individual amino acids present in the sequences can be modified through the use of conventional methods in the process of sample preparation. In another unclaimed example, it is intended that such modified sequences also be captured. For example, SEQ ID NO: 30 contains a cysteine moiety, which can be blocked with a blocking agent in a process of sample preparation, for example, methyl methanethiosulfonate which may result in a sequence in which the cysteine has been blocked from further reacting. Other blocking agents are known in the art and can include iodoacetamide. It should therefore be realized that SEQ ID NO: 30-33 include within their scope modified peptide sequences in which individual amino acids have been modified to include such blocking agents. Persons skilled in the art would recognize that though the sequence will be substantially similar with the addition of the blocking agent, the masses will change accordingly. Such modifications are also applicable to all the sequences of all the allergens disclosed herein.

According to other unclaimed examples, the methods can also be used to identify and quantify other allergens in addition to the allergens disclosed above, such other allergens including low and high molecular weight glutenins, and proteins from mustard, oats, rye, sesame, wheat, brazil nuts, hazelnuts, macadamia nuts, peanuts, pistachio nuts and walnuts. As exemplified above for the allergens such as ovalbumin, lysozyme, casein isoform S1, casein isoform S2, lactoglobulin, and barley, this can also be accomplished by detecting at least one isolated peptide sequence specific to the allergen, for example, one of more of the peptides having the amino acid sequence for the specific allergen previously described or as outlined in Tables 7-19.

According to some unclaimed examples, the method can comprise detecting and/or quantifying the allergen by detecting at least one isolated peptide specific to that allergen, for example, one or more of the peptides having an amino acid sequence of disclosed in Tables 7-19 for a specific allergen. For example, a triple quadrupole mass spectrometer can be used to monitor selected Multiple Reaction Monitoring (MRM) transitions to determine the presence and/or quantity of the allergen-specific peptide in the sample. In some embodiments, the allergen specific peptide can have an amino acid sequence that is described in Tables 7-19, and the method can include monitoring at least one precursor-product ion pair transition having an m/z value specific to that allergen and described in Tables 7-19, and can also include monitoring the fragment ion, specific to that allergen as described, where available in Tables 7-19.

In one unclaimed example, the method can include monitoring multiple precursor-product ion pair transitions associated with each allergen-specific peptide when such a peptide has more than one precursor-product ion pair transition associated with it. By way of example, an allergen specific peptide having an amino acid sequence described in Tables 7 -19 can be identified by monitoring any two (where available) of the precursor-product ion pair transitions found in Tables 7-19 for a specific allergen. In one unclaimed example, and where available, three or more of the identified precursor-product ion pairs can be monitored, where available.

In one unclaimed example, the method for detecting or quantifying other allergens, including low and high molecular weight glutinens, and proteins from mustard, oats, rye, sesame, wheat, brazil nuts, hazelnuts, macadamia nuts, peanuts, pistachio nuts and walnuts, can include detecting multiple allergen-specific peptides. By way of non-limiting example, the allergen can be quantified by using any combination, or all, of the peptides having the amino acid sequences for a specific allergen described in Tables 7-19, identified herein. As discussed above, for each peptide, one or more of the precursor-product ion pair transitions can be monitored.

Tables 7-19 below shows sequences of the peptides determined, according to the present teachings, to be specific to allergens of low and high molecular weight glutenins, and allergens from proteins from mustard, oats, rye, sesame, wheat, brazil nuts, hazelnuts, macadamia nuts, peanuts, pistachio nuts and walnuts, along with their optimal MRM Q1, Q3 transitions and fragment ion. According to various unclaimed examples, these observed peptides and transitions can be used to enable a reliable quantitation of the allergens present in the sample.

For further clarity, according to the present teachings, each of the allergens described in Tables 7-19 shows sequences of the peptides determined for that allergen, along with their optimal MRM Q1, Q3 transitions and fragment ion information. According to various unclaimed examples, these observed peptides and transitions can be used to enable a reliable quantitation of that allergen present in the sample.

**TABLE 7**

| **Glutenin, low MW** | **Q1** | **Q3** | **Fragment Ion** |
|---|---|---|---|
| | **448.2** | **693.3** | **2/y5** |
| **SDCQVMR** | **448.2** | **533.3** | **2/y4** |
| **(SEQ ID NO: 34)** | **448.2** | **808.3** | **2/y6** |
| | **448.2** | **491.2** | **2/b4** |
| **doubly-charged** | **448.2** | **590.2** | **2/b5** |
| **APFASIVASIGGQ** | **609.3** | | **2/b7** |
| **(SEQ ID NO: 35)** | | **686.4** | |
| | **609.3** | | **2/b8** |
| **doubly-charged** | **609.3** | **757.4** | **2/b12** |
| | **609.3** | **1071.6** | **2/y7** |
| | **609.3** | **631.3** | **2/b9** |
| | | **844.5** | |
| **SLVLQTLPTMCNVYVPPYCSTIR** | **904.8** | **993.5** | **3/y8** |
| **(SEQ ID NO: 36)** | **904.8** | **1092.6** | **3/y9** |
| | **904.8** | **953.6** | **3/b9** |
| **triply-charged** | | | **3/b10** |
| | **904.8** | **1084.6** | |
| **SLVLQTLPTMCNVYVPPYCSTIR** | **923.8** | **993.5** | **3/y8** |
| **(SEQ ID NO: 37)** | **923.8** | **1092.6** | **3/y9** |
| **triply-charged** | **923.8** | **1241.6** | **3/b21(2)** |
| **QPFPQQPQQPYPQQPQQPFPQTQQPQQPFPQSK** | **982.7** | **1056.6** | **4/y9** |
| **(SEQ ID NO: 38)** | **982.7** | **1079.5** | **4/b9** |
| | **982.7** | **1118.6** | **4/y19(2)** |
| **quadruple-charged** | **982.7** | **1184.6** | **4/y10** |
| **VSIILPR** | **399.3** | **698.5** | **2/y6** |
| **(SEQ ID NO: 39)** | **399.3** | **498.3** | **2/y4** |
| | | | **2/y5** |
| **doubly-charged** | **399.3** | **611.4** | |
| | **824.5** | **1236.7** | **2/y11** |
| **GIIQPQQPAQLEGIR** | **824.5** | **883.5** | **2/y8** |
| **(SEQ ID NO: 40)** | **824.5** | **1011.6** | **2/y9** |
| | | | **21y10** |
| **doubly-charged** | **824.5** | **1139.6** | **2/b10** |
| | **824.5** | **1061.6** | |
| **AQGLGIIQPQQPAQLEGIR** | **1009.1** | **1236.7** | **2/y11** |
| **(SEQ ID NO: 41)** | **1009.1** | **1134.6** | **2/b11** |
| **doubly-charged** | **1009.1** | **1139.6** | **2/y10** |
| | **879.8** | **1084.7** | **3/y10** |
| **NFLLQQCNHVSLVSSLVSIILPR** | **879.8** | **1183.7** | **3/y11** |
| **(SEQ ID NO: 42)** | **879.8** | **997.6** | **3/y9** |
| | **879.8** | **910.6** | **3/y8** |
| **triply-charged** | **879.8** | **1127.1** | **3/b20(2)** |
| **PSGQVQWPQQQPFPQPQQPFCQQPQR** | **1049.5** | **1182.6** | **3/y19(2)** |
| **(SEQ ID NO: 43)** | **1049.5** | **1060.5** | **3/y8** |
| | **1049.5** | **1136.6** | **3/b10** |
| **triply-charged** | **1049.5** | **1188.6** | **3/y9** |
| | **430.8** | **548.3** | **2/y4** |
| **VNVPLYR** | **430.8** | **647.4** | **2/y5** |
| **(SEQ ID NO: 44)** | **430.8** | **451.3** | **2/y3** |
| **doubly-charged** | **430.8** | **761.4** | **2/y6** |
| | **566.8** | **890.5** | **2/y8** |
| **LEVMTSIALR** | **566.8** | **791.4** | **2/y7** |
| **(SEQ ID NO: 45)** | **566.8** | **660.4** | **2/y6** |
| | | | **2/y9** |
| **doubly-charged** | **566.8** | **1019.6** | |
| | **640.3** | **790.4** | **2/y9** |
| **TTTSVPFGVGTGVG** | **640.3** | **1105.6** | **2/b12** |
| **(SEQ ID NO: 46)** | **640.3** | **890.5** | **2/b9** |
| | | | **2/y8** |
| **doubly-charged** | **640.3** | **693.4** | **2/b11** |
| | **640.3** | **1048.5** | |
| | **675.9** | **861.5** | **2/y10** |
| **TTTSVPFGVGTGVGA** | **675.9** | **890.5** | **2/b9** |
| **(SEQ ID NO: 47)** | **675.9** | **1105.6** | **2/b12** |
| | **675.9** | **1204.6** | **2/b13** |
| **triply-charged** | **675.9** | **764.4** | **2/y9** |
| **TTTSVPFGVGTGVGAY** | **757.4** | **1024.5** | **2/y11** |
| **(SEQ ID N0: 48)** | **757.4** | **890.5** | **2/b9** |
| **doubly-charged** | **757.4** | **780.4** | **2/y9** |
| **ILPTMCSVNVPLYR** | **831.9** | **1107.6** | **2/y9** |
| **(SEQ ID NO: 49)** | **831.9** | **947.5** | **2/y8** |
| **doubly-charged** | **831.9** | **1115.6** | **2/b10** |
| **SQMLQQSSCHVMQQQCCQQLPQIPQQSR** | **875.9** | **953.5** | **4/y8** |
| **(SEQ ID NO: 50)** | **875.9** | **1066.6** | |
| **quadruple-charged** | **875.9** | **1194.7** | **4/y10** |
| **SQMLQQSSCHVMQQQCCQQLPQIPQQSRYEAIR** | **827.4** | **850.0** | **5/y14(2)** |
| **(SEQ ID NO: 51)** | **827.4** | **914.0** | **5/y15/2)** |
| **Quintuply-charged** | **827.4** | **1247.7** | **5/y10** |
| | **594.3** | **927.5** | **2/b10** |
| **APFASIVAGIGGQ** | **594.3** | **814.5** | **2/b9** |
| **(SEQ ID NO: 52)** | **594.3** | **686.4** | **2/b7** |
| | | | **2/b8** |
| **Doubly-charged** | **594.3** | **757.4** | **2/y7** |
| | **594.3** | **601.3** | |
| **APFASIVAGIGGQ** | **622.8** | **871.5** | **2/b9** |
| **(SEQ ID NO: 53)** | **622.8** | **743.4** | **2/b7** |
| | **622.8** | **814.5** | **2/b8** |
| **doubly-charged** | **622.8** | **644.3** | **2/b6** |
| | **622.8** | **984.6** | **2/b10** |
| | **672.4** | **1083.6** | **2/b11** |
| **RAPFASIVAGIGGQ** | **672.4** | **970.6** | **2/b10** |
| **(SEQ ID NO: 54)** | **672.4** | **913.5** | **2/b9** |
| | | | **2/b8** |
| **doubly-charged** | **672.4** | **842.5** | **2/b12** |
| | **672.4** | **1140.7** | |
| **SLVLQTLPSMCNVYVPPECSIMR** | **917.8** | **989.5** | **3/y8** |
| **(SEQ ID NO: 55)** | **917.8** | **1088.5** | **3/y9** |
| **triply-charged** | **917.8** | **1223.6** | **3/by2)** |
| **QQPFPQTQQPQQPFPQQPQQPFPQTQQP** | **1110.2** | **1180.6** | **3/b10** |
| **(SEQ ID NO: 56)** | **1110.2** | **1198.6** | **3/y10** |
| **triply-charged** | **1110.2** | **1242.6** | **3/b21(2)** |
| **QIPEQSRHESIR** | **493.9** | **619.8** | **31y10/2)** |
| **(SEQ ID NO: 57)** | **493.9** | | **3/y5** |
| | **493.9** | **571.3** | **3/y9(2)** |
| **triply-charged** | **493.9** | **506.8** | **3/y8(2)** |
| | **859.5** | **974.5** | **2/y8** |
| **VFLQQQCIPVAMQR** | **859.5** | **1102.6** | **2/y9** |
| **(SEQ ID NO: 58)** | **859.5** | **1230.6** | **2/y10** |
| | | | **2/b8** |
| **doubly-charged** | **859.5** | **1017.5** | **2/b10** |
| | **859.5** | **1213.6** | |
| | **743.1** | **865.5** | **3/y5(2)** |
| **QQQIPVIHPSVLQQLNPCK** | **743.1** | **1000.5** | **3/y8** |
| **(SEQ ID NO: 59)** | **743.1** | **1099.6** | **3/y9** |
| | | | **3/y10** |
| **triply-charged** | **743.1** | **1186.6** | **3/y7(2)** |
| | **743.1** | **986.1** | |

**TABLE 8**

| **Gluten, high** | **Q1** | **Q3** | **Fragment Ion** |
|---|---|---|---|
| **ELQELQER** | **522.8** | **674.4** | **2/y5** |
| **(SEQ ID NO: 60)** | **522.8** | **802.4** | **2/y6** |
| | | | **2/y4** |
| **doubly-charged** | **522.8** | **545.3** | **2/y3** |
| | **522.8** | **432.2** | |
| | **524.3** | **543.2** | **2/b6** |
| | **524.3** | **656.3** | **2/b7** |
| | | | **2/b9** |
| | **524.3** | **814.4** | **2/b8** |
| **LEGGDALSASQ** | | | **2/b10** |
| **(SEQ ID NO: 61)** | **524.3** | **743.4** | **2/y2** |
| | | | **2/y4** |
| **doubly-charged** | **524.3** | **901.4** | |
| | **524.3** | **234.1** | |
| | **524.3** | **392.2** | |
| | **579.4** | **897.6** | **2/y8** |
| | **579.4** | **711.5** | **2/y7** |
| **IFWGIPALLK** | | | **2/b8** |
| **(SEQ ID NO: 62)** | **579.4** | **898.5** | **2/y9** |
| | | | **2/y5** |
| **doubly-charged** | **579.4** | **1044.6** | |
| | **579.4** | **541.4** | |
| | **607.9** | **897.6** | **2/y8** |
| | **607.9** | **711.5** | **2/y7** |
| **IFWGIPALLK** | | | **2/y9** |
| **(SEQ ID NO: 63)** | **607.9** | **1044.6** | **2/y6** |
| | | | **2/y5** |
| **doubly-charged** | **607.9** | **654.5** | |
| | **607.9** | **541.4** | |
| | **657.4** | **697.5** | **2/y6** |
| | **657.4** | **1053.7** | **2/y9** |
| **IFWGIPALLKR** | | | **2/y8** |
| **(SEQ ID NO: 64)** | **657.4** | **867.6** | **2/y7** |
| | | | **2/y10** |
| **doubly-charged** | **657.4** | **810.6** | |
| | **657.4** | **1200.7** | |
| | **682.3** | **705.3** | **2/y5** |
| | **682.3** | **833.4** | **2/y6** |
| **GEASEQLQCER** | | | **2/y9** |
| **(SEQ ID NO: 65)** | **682.3** | **1177.5** | **2/b7** |
| | | | **2/y4** |
| **doubly-charged** | **682.3** | **772.4** | |
| | **682.3** | **592.3** | |
| | **685.9** | **1053.7** | **2/y9** |
| **IFWGIPALLKR** | **685.9** | **697.5** | **2/y6** |
| **(SEQ ID NO: 66)** | | | **2/y8** |
| | **685.9** | **867.6** | **2/y10** |
| **doubly-charged** | **685.9** | **1200.7** | |
| **EGEASEQLQCER** | **718.3** | **1049.5** | **2/y8** |
| **(SEQ ID NO: 67)** | **718.3** | **833.4** | **2/y6** |
| | | | **2/y7** |
| **doubly-charged** | | | **2/y9** |
| | **718.3** | **962.4** | **2/y4** |
| | **718.3** | **1120.5** | |
| | **718.3** | **592.3** | |
| | **729.4** | **1017.5** | **2/y9** |
| | **729.4** | **946.5** | **2/y8** |
| **AQQLAAQLPAMCR** | | | **2/y7** |
| **(SEQ ID NO: 68)** | **729.4** | **875.4** | **2/y10** |
| | | | **2/y6** |
| **doubly-charged** | **729.4** | **1130.6** | |
| | **729.4** | **747.4** | |
| | **458.8** | **730.4** | **2/y7** |
| | **458.8** | **560.3** | **2/y5** |
| **SVAVSQVAR** | | | **2/y6** |
| **(SEQ ID NO: 69)** | **458.8** | **659.4** | **2/y4** |
| | | | **2/y8** |
| **doubly-charged** | **458.8** | **473.3** | |
| | **458.8** | **829.5** | |
| | **557.3** | **886.5** | **2/y8** |
| **QVVDQQLAGR** | **557.3** | **787.4** | **2/y7** |
| **(SEQ ID NO: 70)** | | | **2/y6** |
| **doubly-charged** | **557.3** | **672.4** | **2/y9** |
| | **557.3** | **985.5** | |
| | **661.3** | **822.4** | **2/y7** |
| | **661.3** | **951.4** | **2/y8** |
| **ELQESSLEACR** | | | **2/y6** |
| **(SEQ ID NO: 71)** | **661.3** | **735.4** | **2/y9** |
| | | | **2/y4** |
| **doubly-charged** | **661.3** | **1079.5** | |
| | **661.3** | **535.2** | |
| | **735.4** | **1142.6** | **2/y10** |
| **AQQPATQLPTVCR** | **735.4** | **745.4** | **2/y6** |
| **(SEQ ID NO: 72)** | **735.4** | **974.5** | **2/y8** |
| | **735.4** | **873.5** | **2/y7** |
| **doubly-charged** | **735.4** | **1045.6** | **2/y9** |
| | **735.4** | **632.3** | **2/y5** |
| | **712.7** | **822.4** | **3/y7** |
| | **712.7** | **951.4** | **3/y8** |
| **QLQCERELQESSLEACR** | | | |
| **(SEQ ID NO: 73)** | **712.7** | **735.4** | |
| | | | **3/y6** |
| **triply-charged** | **712.7** | **947.9** | **3/y15/2)** |
| | **712.7** | **883.9** | **3/y14(2)** |
| **QVVDQQLAGRLPWSTGLQMR** | **761.7** | **1075.5** | **3/y9** |
| **(SEQ ID NO: 74)** | | | **3/y18(2)** |
| | **761.7** | **1028.5** | **3/y14(2)** |
| **triply-charged** | **761.7** | **793.4** | **3/y17(2)** |
| | | | **3/y15/2)** |
| | **761.7** | **979.0** | |
| | **761.7** | **857.5** | |
| | **1048.3** | **1236.6** | **4/y11** |
| | **1048.3** | **1120.5** | **4/b10** |
| | | | **4/y10** |
| **QQPVQGQQPEQGQQPGQWQQGYYPTSPQQLGQGQQPR** | **1048.3** | **1139.6** | **4/b8** |
| **(SEQ ID NO: 75)** | | | **4/y7** |
| | **1048.3** | **894.4** | **4/y14(2)** |
| **quadruply-charged** | **1048.3** | **770.4** | |
| | **1048.3** | **761.4** | |
| | **966.7** | **1166.6** | **4/b10** |
| | **966.7** | **995.5** | **4/y18(2)** |
| **QGYYPTSLQQPGQGQQIGQGQQGYYPTSPQHTGQR** | | | **4/y10** |
| **(SEQ ID NO: 76)** | **966.7** | **1108.6** | **4/y22(2)** |
| | | | **4/b9** |
| **quadruply-charged** | **966.7** | **1208.6** | |
| | **966.7** | **1038.5** | |
| **QWLQPR** | **414.2** | **513.3** | **2/y4** |
| **(SEQ ID NO: 77)** | **414.2** | **428.2** | **2/b3** |
| | | | |
| **doubly-charged** | **414.2** | **699.4** | |
| | **537.3** | **656.3** | **2/b7** |
| **LEGGDALLASQ** | **537.3** | **543.2** | **2/b6** |
| **(SEQ ID NO: 78)** | | | **2/b8** |
| | **537.3** | **769.4** | **2/b9** |
| **doubly-charged** | **537.3** | **840.5** | |
| | **565.8** | **713.4** | **2/b7** |
| **LEGGDALLASQ** | **565.8** | **600.3** | **2/b6** |
| **(SEQ ID NO: 79)** | | | **2/b8** |
| | **565.8** | **826.4** | **2/b9** |
| **doubly-charged** | **565.8** | **897.5** | |

**Table 9**

| **Mustard Proteins** | **Q1** | **Q3** | **Fragment Ion** |
|---|---|---|---|
| **PAGPFR** | **322.7** | **476.3** | **2/y4** |
| **(SEQ ID NO: 80)** | **322.7** | **547.3** | **2/y5** |
| | **322.7** | **470.2** | **2/b5** |
| **doubly-charged** | **322.7** | **419.2** | **2/y3** |
| **PAGPFRIPK** | **491.8** | **757.5** | **2/y6** |
| **(SEQ ID NO: 81)** | **491.8** | **660.4** | **2/y5** |
| | **491.8** | **814.5** | **2/y7** |
| **doubly-charged** | **491.8** | **885.5** | **2/y8** |
| **IYQTATHLPK** | **586.3** | **666.4** | **2/y6** |
| **(SEQ ID NO: 82)** | **586.3** | **767.4** | **2/y7** |
| | **586.3** | **895.5** | **2/y8** |
| **doubly-charged** | | | |
| **QVSVCPFK** | **482.8** | **650.3** | **2/y5** |
| **(SEQ ID NO: 83)** | **482.8** | **737.4** | **2/y6** |
| | **482.8** | **836.4** | **2/y7** |
| **doubly-charged** | **482.8** | **818.4** | **2/b7** |
| **QVSVCPFKK** | **546.8** | **778.4** | **2/y6** |
| **(SEQ ID NO: 84)** | **546.8** | **865.5** | **2/y7** |
| | **546.8** | **964.5** | **2/y8** |
| **doubly-charged** | | | |
| **QVSVCPFKK** | **364.9** | **519.3** | **3/y4** |
| **(SEQ ID NO: 85)** | | | |
| | | | |
| **triply-charged** | | | |
| **PAGPFGIPK** | **442.3** | **658.4** | **2/y6** |
| **(SEQ ID NO: 86)** | **442.3** | **715.4** | **2/y7** |
| | **442.3** | **786.5** | **2/y8** |
| **doubly-charged** | **442.3** | **737.4** | **2/b8** |
| **QQLEQQGQQGPHLQHVISR** | **737.7** | **1143.6** | **3/y10** |
| **(SEQ ID NO: 87)** | **737.7** | **989.6** | **3/y8** |
| | **737.7** | **1086.6** | **3/y9** |
| **triply-charged** | | | |
| **QQLEQQGQQGPHLQHVISR** | **553.5** | **1086.6** | **4/y9** |
| **(SEQ ID NO: 88)** | | | |
| | | | |
| **quadruply-charged** | | | |
| **IYQTATHLPR** | **600.3** | **694.4** | **2/y6** |
| **(SEQ ID NO: 89)** | **600.3** | **795.5** | **2/y7** |
| | **600.3** | **923.5** | **2/y8** |
| **doubly-charged** | | | |
| **IYQTATHLPR** | **400.6** | **694.4** | **3/y6** |
| **(SEQ ID NO: 90)** | | | |
| | | | |
| **triply-charged** | | | |
| **QVSVCPFQK** | **546.8** | **778.4** | **2/y6** |
| **(SEQ ID NO: 91)** | **546.8** | **865.4** | **2/y7** |
| | **546.8** | **964.5** | **2/y8** |
| **doubly-charged** | | | |
| **QVSVCPFQK** | **364.9** | **519.3** | **3/y4** |
| **(SEQ ID NO: 92)** | | | |
| | | | |
| **triply-charged** | | | |
| **VCNIPQVSVCPFK** | **774.4** | **1061.5** | **2/y9** |
| **(SEQ ID NO: 93)** | **774.4** | **964.5** | **2/y8** |
| | **774.4** | **1174.6** | **2/y10** |
| **doubly-charged** | | | |
| **VCNIPQVSVCPFK** | **516.6** | **964.5** | **3/y8** |
| **(SEQ ID NO: 94)** | | | |
| | | | |
| **triply-charged** | | | |
| **VCNIPQVSVCPFKK** | **838.4** | **1189.6** | **2/y10** |
| **(SEQ ID NO: 95)** | **838.4** | **964.5** | **2/y8** |
| | **838.4** | **1092.6** | **2/y9** |
| **doubly-charged** | | | |
| **VCNIPQVSVCPFKK** | **559.3** | **964.5** | **3/y8** |
| **(SEQ ID NO: 96)** | | | |
| **triply-charged** | | | |
| **QQLGQQGQQGPQVQHVISR** | **1058.6** | **1120.6** | **2/y10** |
| **(SEQ ID NO: 97)** | **1058.6** | **1248.7** | **2/y11** |
| | **1058.6** | **1063.6** | **2/y9** |
| **doubly-charged** | | | |
| **QQLGQQGQQGPQVQHVISR** | **706.0** | **1063.6** | **3/y9** |
| **(SEQ ID NO: 98)** | | | |
| | | | |
| **triply-charged** | | | |
| **IYQTATHLPR** | **600.3** | **1086.6** | **2/y9** |
| **(SEQ ID NO: 99)** | | | |
| **doubly-charged** | | | |
| **AGPFR** | **274.2** | **419.2** | **2/y3** |
| **(SEQ ID NO: 100)** | **274.2** | **476.3** | **2/y4** |
| | **274.2** | **322.2** | **2/y2** |
| **doubly-charged** | **274.2** | **373.2** | **2/b4** |
| **AVKQQIR** | **421.8** | **672.4** | **2/y5** |
| **(SEQ ID NO: 101)** | **421.8** | **771.5** | **2/y6** |
| | **421.8** | **668.4** | **2/b6** |
| **doubly-charged** | **421.8** | **555.3** | **2/b5** |
| **QQGQQQGQQGQQLQHEISR** | **736.0** | **882.5** | **3/y7** |
| **(SEQ ID NO: 102)** | **736.0** | **1010.5** | **3/y8** |
| | **736.0** | **1138.6** | **3/y9** |
| **triply-charged** | **736.0** | **1195.6** | **3/y10** |
| **VCNIPR** | **379.7** | **499.3** | **2/y4** |
| **(SEQ ID NO: 103)** | **379.7** | **659.3** | **2/y5** |
| | **379.7** | **584.3** | **2/b5** |
| **doubly-charged** | **379.7** | **487.2** | **2/b4** |
| **VSICPFQK** | **489.8** | **519.3** | **2/y4** |
| **(SEQ ID NO: 104)** | **489.8** | **679.3** | **2/y5** |
| | **489.8** | **792.4** | **2/y6** |
| **doubly-charged** | | | |
| **VSICPFQK** | **326.8** | **519.3** | **3/y4** |
| **(SEQ ID NO: 105)** | | | |
| | | | |
| **triply-charged** | | | |
| **EFRQAQHLR** | **592.8** | **1055.6** | **2/y8** |
| **(SEQ ID N0: 106)** | **592.8** | **752.4** | **2/y6** |
| | **592.8** | **908.5** | **2/y7** |
| **doubly-charged** | | | |
| **EFRQAQHLR** | **395.6** | **752.4** | **3/y6** |
| **(SEQ ID NO: 107)** | | | |
| | | | |
| **triply-charged** | | | |
| **QAQHLR** | **376.7** | **553.3** | **2/y4** |
| **(SEQ ID NO: 108)** | **376.7** | **624.4** | **2/y5** |
| | **376.7** | **578.3** | **2/b5** |
| **doubly-charged** | **376.7** | **465.2** | **2/b4** |
| **ACQQWLHR** | **549.8** | **739.4** | **2/y5** |
| **(SEQ ID NO: 109)** | **549.8** | **867.5** | **2/y6** |
| | **549.8** | **1027.5** | **2/y7** |
| **doubly-charged** | **549.8** | **924.4** | **2/b7** |
| | | | |
| **QQVRQQGHQQQMQHVISR** | **739.4** | **1218.6** | **3/b10** |
| **(SEQ ID NO: 110)** | **739.4** | **998.5** | **3/y8** |
| | **739.4** | **1126.6** | **3/y9** |
| **triply-charged** | | | |
| **QQVRQQGHQQQMQHVISR** | **554.8** | **998.5** | **4/y8** |
| **(SEQ ID NO: 111)** | | | |
| | | | |
| **quadruply-charged** | | | |
| **IYQTATHLPR** | **600.3** | **694.4** | **2/y6** |
| **(SEQ ID NO: 112)** | **600.3** | **795.5** | **2/y7** |
| | **600.3** | **923.5** | **2/y8** |
| **doubly-charged** | **600.3** | **1086.6** | **2/y9** |

**Table 10**

| **Oats Proteins** | **Q1** | **Q3** | **Fragment Ion** |
|---|---|---|---|
| **QFLVQQCSPVAVVPFLR** | **989.0** | **997.6** | **2/y9** |
| **(SEQ ID NO: 113)** | **989.0** | **1084.7** | **2/y10** |
| | **989.0** | **1233.7** | **2/y11** |
| **doubly-charged** | | | |
| **SQILQQSSCQVMR** | **777.4** | **984.4** | **2/y8** |
| **(SEQ ID NO: 114)** | **777.4** | **1112.5** | **2/y9** |
| | **777.4** | **1225.6** | **2/y10** |
| **doubly-charged** | | | |
| **QLEQIPEQLR** | **627.4** | **642.4** | **2/y5** |
| **(SEQ ID NO: 115)** | **627.4** | **1012.5** | **2/y8** |
| | | | |
| **doubly-charged** | | | |
| **QLEQIPEQLR (SEQ ID NO: 116)** | **418.6** | **642.4** | **3/y5** |
| | | | |
| **triply-charged** | | | |

**Table 11**

| **Rye Proteins** | **Q1** | **Q3** | **Fragment Ion** |
|---|---|---|---|
| **NVLLQQCSPVALVSSLR** | **937.0** | **1177.6** | **2/y11** |
| **(SEQ ID NO: 117)** | | | |
| | | | |
| **doubly-charged** | | | |
| **NVLLQQCSPVALVSSLR** | **625.0** | **941.6** | **3/y9** |
| **(SEQ ID NO: 118)** | **625.0** | **1177.6** | **3/y11** |
| | | | |
| **triply-charged** | | | |
| **EGVQILLPQSHQQHVGQGAL-AQVQGIIQPQQLSQLEWR** | **851.7** | **1199.7** | **5/y10** |
| **(SEQ ID NO: 119)** | **851.7** | **1071.6** | **5/y9** |
| | **851.7** | **1210.2** | **5/b23(2)** |
| **quintuply-charged** | | | |
| **SLVLQNLPTMCNVYVPR (SEQ ID NO: 120)** | **997.0** | **1225.6** | **2/y10** |
| | | | |
| **doubly-charged** | | | |
| **SLVLQNLPTMCNVYVPR** | **665.0** | **1225.6** | **3/y10** |
| **(SEQ ID NO: 121)** | **665.0** | **1128.5** | **3/y9** |
| **triply-charged** | | | |
| **QCSTIQAPFASIVTGIVGH** | **988.0** | **1100.6** | **2/y11** |
| **(SEQ ID NO: 122)** | **988.0** | **1197.7** | **2/y12** |
| | | | |
| **doubly-charged** | | | |
| **QCSTIQAPFASIVTGIVGH** | **659.0** | **1197.7** | **3/y12** |
| **(SEQ ID NO: 123)** | | | |
| | | | |
| **triply-charged** | | | |

**Table 12**

| **Sesame Proteins** | **Q1** | **Q3** | **Fragment Ion** |
|---|---|---|---|
| **ISGAQPSLR** | **464.8** | **472.3** | **2/y4** |
| **(SEQ ID NO: 124)** | **464.8** | **728.4** | **2/y7** |
| | **464.8** | **815.4** | **2/y8** |
| **doubly-charged** | | | |
| **LVYIER** | **396.7** | **679.4** | **2/y5** |
| **(SEQ ID NO: 125)** | **396.7** | **417.3** | **2/y3** |
| | **396.7** | **579.8** | |
| **doubly-charged** | | | |
| **AFDAELLSEAFNVPQETIR** | **717.4** | **743.4** | **3/y6** |
| **(SEQ ID NO: 126)** | **717.4** | **956.5** | **3/y8** |
| | **717.4** | **372.4** | **3/y6(2)** |
| **triply-charged** | | | |
| **GLIVMAR** | **380.2** | **589.4** | **2/y5** |
| **(SEQ ID NO: 127)** | **380.2** | **476.3** | **2/y4** |
| | **380.2** | **377.2** | **2/y3** |
| **doubly-charged** | | | |
| **EADIFSR** | **419.2** | **522.3** | **2/y4** |
| **(SEQ ID NO: 128)** | **419.2** | **637.3** | **2/y5** |
| | **419.2** | **409.2** | **2/y3** |
| **doubly-charged** | | | |
| **SPLAGYTSVIR** | **582.3** | **795.4** | **2/y7** |
| **(SEQ ID NO: 129)** | **582.3** | **866.5** | **2/y8** |
| | **582.3** | **979.6** | **2/y9** |
| **doubly-charged** | | | |
| **AMPLQVITNSYQISPNQAQALK** | **805.8** | **869.5** | **3/y8** |
| **(SEQ ID NO: 130)** | **805.8** | **1069.6** | **3/y10** |
| | **805.8** | **956.5** | **3/y9** |
| **triply-charged** | | | |
| **HCMQWMR** | **519.2** | **751.3** | **2/y5** |
| **(SEQ ID NO: 131)** | **519.2** | **900.3** | **2/y6** |
| | **519.2** | **620.3** | **2/y4** |
| **doubly-charged** | | | |
| **MCGMSYPTECR** | **685.2** | **901.4** | **2/y7** |
| **(SEQ ID NO: 132)** | **685.2** | **1089.4** | **2/y9** |
| | **685.2** | **1238.4** | **2/y10** |
| **doubly-charged** | | | |

**Table 13**

| **Wheat Proteins** | **Q1** | **Q3** | **Fragment Ion** |
|---|---|---|---|
| **SVAVSQVAR** | **458.8** | **730.4** | **2/y7** |
| **(SEQ ID NO: 133)** | **458.8** | **560.3** | **2/y5** |
| | | | |
| **doubly-charged** | | | |
| **EHGAQEGQAGTGAFPR** | **538.3** | **547.3** | **3/y5** |
| **(SEQ ID NO: 134)** | **538.3** | **776.4** | **3/y8** |
| | **538.3** | **705.4** | **3/y7** |
| **triply-charged** | | | |
| **QWDQQLAGR** | **557.3** | **886.5** | **2/y8** |
| **(SEQ ID NO: 135)** | **557.3** | **787.4** | **2/y7** |
| | | | |
| **doubly-charged** | | | |
| **IFWGIPALLK** | **579.4** | **897.6** | **2/y8** |
| **(SEQ ID NO: 136)** | **579.4** | **711.5** | **2/y7** |
| | | | |
| **doubly-charged** | | | |
| **LPWSTGLQMR** | **594.8** | **792.4** | **2/y7** |
| **(SEQ ID NO: 137)** | **594.8** | **978.5** | **2/y8** |
| | **594.8** | **538.9** | **2/y9(2)** |
| **doubly-charged** | | | |
| **YDPTAYNTILR** | **663.8** | **850.5** | **2/y7** |
| **(SEQ ID NO: 138)** | **663.8** | **779.4** | **2/y6** |
| | | | |
| **doubly-charged** | | | |

**Table 14**

| **Brazil Proteins** | **Q1** | **Q3** | **Fragment Ion** |
|---|---|---|---|
| **QQMLSHCR** | **524.7** | **661.3** | **2/y5** |
| **(SEQ ID NO: 139)** | **524.7** | **792.3** | **2/y6** |
| | **524.7** | **548.2** | **2/y4** |
| **doubly-charged** | | | |
| **GMEPHMSECCEQLEGMDESCR** | **847.0** | **1085.4** | **3/y9** |
| **(SEQ ID NO: 140)** | **847.0** | **843.3** | **3/y7** |
| | **847.0** | **972.3** | **3/y8** |
| **triply-charged** | | | |
| **MMMMR** | **350.1** | **568.2** | **2/y4** |
| **(SEQ ID NO: 141)** | **350.1** | **437.2** | **2/y3** |
| | **350.1** | **306.5** | |
| **doubly-charged** | | | |
| **MQQEEMQPR** | **588.8** | **660.3** | **2/y5** |
| **(SEQ ID NO: 142)** | **588.8** | **789.4** | **2/y6** |
| | **588.8** | **917.4** | **2/y7** |
| **doubly-charged** | | | |
| **LAENIPSR** | **450.3** | **586.3** | **2/y5** |
| **(SEQ ID NO: 143)** | **450.3** | **715.4** | **2/y6** |
| | **450.3** | **786.4** | **2/y7** |
| **doubly-charged** | | | |
| **CNLSPMR** | **433.7** | **603.3** | **2/y5** |
| **(SEQ ID NO: 144)** | **433.7** | **490.2** | **2/y4** |
| | **433.7** | **403.2** | **2/y3** |
| **doubly-charged** | | | |
| **QQQLNHCR** | **536.7** | **688.3** | **2/y5** |
| **(SEQ ID NO: 145)** | **536.7** | **816.4** | **2/y6** |
| | **536.7** | **575.2** | **2/y4** |
| **doubly-charged** | | | |
| **MDEMCR** | **415.6** | **584.2** | **2/y4** |
| **(SEQ ID NO: 146)** | **415.6** | **699.2** | **2/y5** |
| | **415.6** | **507.2** | **2/b4** |
| **doubly-charged** | **415.6** | **455.2** | **2/y3** |
| **CNLSPQR** | **432.2** | **600.4** | **2/y5** |
| **(SEQ ID NO: 147)** | **432.2** | **487.3** | **2/y4** |
| | **432.2** | **400.2** | **2/y3** |
| **doubly-charged** | | | |
| **CAGVAALR** | **403.7** | **529.4** | **2/y5** |
| **(SEQ ID NO: 148)** | **403.7** | **586.4** | **2/y6** |
| | **403.7** | **657.4** | **2/y7** |
| **doubly-charged** | **403.7** | **519.2** | **2/b6** |
| | **403.7** | **430.3** | **2/y4** |
| **LYYVTQGR** | **500.3** | **723.4** | **2/y6** |
| **(SEQ ID NO: 149)** | **500.3** | **886.4** | **2/y7** |
| | **500.3** | **461.3** | **2/y4** |
| **doubly-charged** | | | |
| **HFFLAGNIQR** | **601.8** | **771.5** | **2/y7** |
| **(SEQ ID NO: 150)** | **601.8** | **918.5** | **2/y8** |
| | **601.8** | **658.4** | **2/y6** |
| **doubly-charged** | | | |
| **GGQQILADNVFK** | **645.4** | **806.4** | **2/y7** |
| **(SEQ ID NO: 151)** | **645.4** | **919.5** | **2/y8** |
| | **645.4** | **693.4** | **2/y6** |
| **doubly-charged** | | | |
| **GFNMEALADVLGFGMDTETAR** | **749.0** | **880.4** | **3/y8** |
| **(SEQ ID NO: 152)** | **749.0** | **1084.5** | **3/y10** |
| | **749.0** | **692.3** | **3/y6** |
| **triply-charged** | | | |
| **GVLYENAMMAPLWR** | **825.9** | **1089.5** | **2/y9** |
| **(SEQ ID NO: 153)** | **825.9** | **904.5** | **2/y7** |
| | **825.9** | **571.3** | **2/y4** |
| **doubly-charged** | | | |
| **GIPVGVLANAYR** | **615.4** | **863.5** | **2/y8** |
| **(SEQ ID NO: 154)** | **615.4** | **962.5** | **2/y9** |
| | **615.4** | **707.4** | **2/y6** |
| **doubly-charged** | | | |
| **DEAVLFQPGSR** | **609.8** | **804.4** | **2/y7** |
| **(SEQ ID NO: 155)** | **609.8** | **903.5** | **2/y8** |
| | **609.8** | **691.4** | **2/y6** |
| **doubly-charged** | | | |

**Table 15**

| **Hazelnuts Proteins** | **Q1** | **Q3** | **Fragment Ion** |
|---|---|---|---|
| **YFGECNLDR** | **581.7** | **666.3** | **2/y5** |
| **(SEQ ID NO: 156)** | **581.7** | **852.3** | **2/y7** |
| | **581.7** | **999.4** | **2/y8** |
| **doubly-charged** | | | |
| **LNALEPTNR** | **514.3** | **729.4** | **2/y6** |
| **(SEQ ID NO: 157)** | **514.3** | **800.4** | **2/y7** |
| | **514.3** | **914.5** | **2/y8** |
| **doubly-charged** | | | |
| **TIEPNGLLLPQYSNAPELIYIER** | **881.8** | **1032.6** | **3/y8** |
| **(SEQ ID NO: 158)** | **881.8** | **580.3** | **3/y4** |
| | **881.8** | **806.5** | **3/y6** |
| **triply-charged** | | | |
| **HFYLAGNPDDEHQR** | **566.9** | **448.8** | **3/y7(2)** |
| **(SEQ ID NO: 159)** | **566.9** | **684.3** | **3/y5** |
| | **566.9** | **799.3** | |
| **triply-charged** | | | **3/y6** |
| **QGQQQFGQR** | **538.8** | **763.4** | **2/y6** |
| **(SEQ ID NO: 160)** | **538.8** | **948.5** | **2/y8** |
| | **538.8** | **635.3** | **2/y5** |
| **doubly-charged** | | | |
| **QEWER** | **374.2** | **619.3** | **2/y4** |
| **(SEQ ID NO: 161)** | **374.2** | **490.2** | **2/y3** |
| | **374.2** | **304.2** | **2/y2** |
| **doubly-charged** | | | |
| **ADIYTEQVGR** | **576.3** | **689.4** | **2/y6** |
| **(SEQ ID NO: 162)** | **576.3** | **852.4** | **2/y7** |
| | **576.3** | **588.3** | **2/y5** |
| **doubly-charged** | | | |
| **INTVNSNTLPVLR** | **720.9** | **899.5** | **2/y8** |
| **(SEQ ID NO: 163)** | **720.9** | **1013.6** | **2/y9** |
| | **720.9** | **484.3** | **2/y4** |
| **doubly-charged** | | | |
| **WLQLSAER** | **501.8** | **703.4** | **2/y6** |
| **(SEQ ID NO: 164)** | **501.8** | **816.5** | **2/y7** |
| | **501.8** | **575.3** | **2/y5** |
| **doubly-charged** | | | |
| **QGQVLTIPQNFAVAK** | **807.5** | **874.5** | **2/y8** |
| **(SEQ ID NO: 165)** | **807.5** | **987.6** | **2/y9** |
| **doubly-charged** | **807.5** | **1088.6** | **2/y10** |
| | | | |
| **AESEGFEWVAFK** | **700.3** | **983.5** | **2/y8** |
| **(SEQ ID NO: 166)** | **700.3** | **464.3** | **2/y4** |
| | **700.3** | **365.2** | **2/y3** |
| **doubly-charged** | | | |
| **TNDNAQISPLAGR** | **678.9** | **841.5** | **2/y8** |
| **(SEQ ID NO: 167)** | **678.9** | **600.4** | **2/y6** |
| | **678.9** | **713.4** | **2/y7** |
| **doubly-charged** | | | |
| **ALPDDVLANAFQISR** | **815.4** | **906.5** | **2/y8** |
| **(SEQ ID NO: 168)** | **815.4** | **835.4** | **2/y7** |
| | **815.4** | **723.6** | **2/y13(2)** |
| **doubly-charged** | | | |
| **QETTLVR** | **423.7** | **589.4** | **2/y5** |
| **(SEQ ID NO: 169)** | **423.7** | **718.4** | **2/y6** |
| | **423.7** | **488.3** | **2/y4** |
| **doubly-charged** | | | |
| **VEEIDHANFK** | **601.3** | **973.5** | **2/y8** |
| **(SEQ ID NO: 170)** | **601.3** | **616.3** | **2/y5** |
| | **601.3** | **731.4** | **2/y6** |
| **doubly-charged** | | | |
| **AVEAYLLAHPDAYC** | **791.4** | **935.4** | **2/y8** |
| **(SEQ ID NO: 171)** | **791.4** | **1211.5** | **2/y10** |
| | | | |
| **doubly-charged** | | | |
| **AVEAYLLAHPDAYC** | **527.9** | **614.2** | **3/y5** |
| **(SEQ ID NO: 172)** | | | |
| | | | |
| **triply-charged** | | | |

**Table 16**

| **Macadamia Proteins** | **Q1** | **Q3** | **Fragment Ion** |
|---|---|---|---|
| **QCMQLETSGQMR** | **729.3** | **921.5** | **2/y8** |
| **(SEQ ID NO: 173)** | **729.3** | **1049.5** | **2/y9** |
| | | | |
| **doubly-charged** | | | |
| **CVSQCDK** | **437.7** | **626.2** | **2/y5** |
| **(SEQ ID NO: 174)** | **437.7** | **725.3** | **2/y6** |
| | | | |
| **doubly-charged** | | | |
| **FEEDIDWSK** | **584.8** | **892.4** | **2/y7** |
| **(SEQ ID NO: 175)** | **584.8** | **763.4** | **2/y6** |
| | | | |
| **doubly-charged** | | | |
| **HMQICQQR** | **545.2** | **821.4** | **2/y6** |
| **(SEQ ID NO: 176)** | **545.2** | **952.4** | **2/y7** |
| | | | |
| **doubly-charged** | | | |
| **HCEQQEPR** | **536.7** | **786.4** | **2/y6** |
| **(SEQ ID NO: 177)** | **536.7** | **935.4** | **2/y7** |
| | | | |
| **doubly-charged** | | | |
| **LQYQCQR** | **492.7** | **743.3** | **2/y5** |
| **(SEQ ID NO: 178)** | **492.7** | **580.2** | **2/y4** |
| | | | |
| **doubly-charged** | | | |
| **EGVIIR** | **343.7** | **500.4** | **2/y4** |
| **(SEQ ID NO: 179)** | **343.7** | **401.3** | **2/y3** |
| | | | |
| **doubly-charged** | | | |
| **ESEFDR** | **391.7** | **566.3** | **2/y4** |
| **(SEQ ID NO: 180)** | **391.7** | **437.2** | **2/y3** |
| | | | |
| **doubly-charged** | | | |
| **QQYQCQR** | **500.2** | **743.3** | **2/y5** |
| **(SEQ ID NO: 181)** | **500.2** | **580.2** | **2/y4** |
| | | | |
| **doubly-charged** | | | |

**Table 17**

| **Peanut Proteins** | **Q1** | **Q3** | **Fragment Ion** |
|---|---|---|---|
| **NLPQQCGLR** | **543.3** | **858.4** | **2/y7** |
| **(SEQ ID NO: 182)** | **543.3** | **429.7** | **2/y7(2)** |
| | **543.3** | **633.3** | **2/y5** |
| **doubly-charged** | | | |
| **NNPFYFPSR** | **571.3** | **913.5** | **2/y7** |
| **(SEQ ID NO: 183)** | **571.3** | **669.3** | **2/y5** |
| | **571.3** | **506.3** | **2/y4** |
| **doubly-charged** | | | |
| **TANDLNLLILR** | **628.4** | **854.6** | **2/y7** |
| **(SEQ ID NO: 184)** | **628.4** | **741.5** | **2/y6** |
| | **628.4** | **514.4** | **2/y4** |
| **doubly-charged** | **628.4** | **969.6** | **2/y8** |
| **DLAFPGSGEQVEK** | **688.8** | **833.4** | **2/y8** |
| **(SEQ ID NO: 185)** | **688.8** | **930.5** | **2/y9** |
| | **688.8** | **1077.5** | **2/y10** |
| **doubly-charged** | | | |
| **SPDIYNPQAGSLK** | **695.4** | **700.4** | **2/y7** |
| **(SEQ ID NO: 186)** | **695.4** | **814.4** | **2/y8** |
| | **695.4** | **977.5** | **2/y9** |
| **doubly-charged** | | | |
| **VLLEENAGGEQEER** | **786.9** | **804.4** | **2/y7** |
| **(SEQ ID NO: 187)** | **786.9** | **989.4** | **2/y9** |
| | **786.9** | **1118.5** | **2/y10** |
| **doubly-charged** | | | |

**Table 18**

| **Pistachio Proteins** | **Q1** | **Q3** | **Fragment Ion** |
|---|---|---|---|
| **CAGVAVAR** | **396.7** | **515.3** | **2y5** |
| **(SEQ ID NO: 188)** | **396.7** | **572.4** | **2y6** |
| | **396.7** | **416.3** | **2y4** |
| **doubly-charged** | | | |
| **HTIQPNGLR** | **518.3** | **556.3** | **2y5** |
| **(SEQ ID NO: 189)** | **518.3** | **797.5** | **2y7** |
| **doubly-charged** | | | |
| **ILAEVFQVEQSLVK** | **802.0** | **1077.6** | **2y9** |
| **(SEQ ID NO: 190)** | **802.0** | **802.5** | **2y7** |
| | **802.0** | **930.5** | **2y8** |
| **doubly-charged** | | | |
| **GFESEEESEYER** | **745.8** | **1157.5** | **2y9** |
| **(SEQ ID NO: 191)** | **745.8** | **941.4** | **2y7** |
| | **745.8** | **683.3** | **2y5** |
| **doubly-charged** | | | |
| **SDIYTPEVGR** | **568.8** | **821.4** | **2y7** |
| **(SEQ ID NO: 192)** | **568.8** | **557.3** | **2y5** |
| | **568.8** | **658.4** | **2y6** |
| **doubly-charged** | | | |
| **ITSLNSLNLPILK** | **713.4** | **1011.6** | **2y9** |
| **(SEQ ID NO: 193)** | **713.4** | **470.3** | **2y4** |
| **doubly-charged** | | | |
| **WLQLSAER** | **501.8** | **703.4** | **2y6** |
| **(SEQ ID NO: 194)** | **501.8** | **816.5** | **2y7** |
| | **501.8** | **575.3** | **2y5** |
| **doubly-charged** | | | |
| **FEWISFK** | **478.8** | **680.4** | **2y5** |
| **(SEQ ID NO: 195)** | **478.8** | **809.4** | **2y6** |
| | **478.8** | **381.2** | **2y3** |
| **doubly-charged** | | | |
| **AMISPLAGSTSVLR** | **701.9** | **790.4** | **2y8** |
| **(SEQ ID NO: 196)** | **701.9** | **903.5** | **2y9** |
| | **701.9** | **1087.6** | **2y11** |
| **doubly-charged** | | | |
| **AMPEEVLANAFQISR** | **838.4** | **1019.6** | **2y9** |
| **(SEQ ID NO: 197)** | **838.4** | **835.4** | **2y7** |
| | **838.4** | **906.5** | **2y8** |
| **doubly-charged** | | | |
| **FQTQC[MSH]QIQNLNALEPK** | **961.0** | **1026.6** | **2y9** |
| **(SEQ ID NO: 198)** | **961.0** | **898.5** | **2y8** |
| | **961.0** | **671.4** | **2y6** |
| **doubly-charged** | | | |
| **IESEAGVTEFWDQNEEQLQC[MSH]ANVAVFR** | **790.4** | **1053.5** | **4y9** |
| **(SEQ ID NO: 199)** | **790.4** | **492.3** | **4y4** |
| | **790.4** | **925.4** | **4y8** |
| **doubly-charged** | | | |
| **LVLVALADVGNSENQLDQYLR** | **777.4** | **694.4** | **3y5** |
| **(SEQ ID NO: 200)** | **777.4** | **807.4** | **3y6** |
| | **777.4** | **579.3** | **3y4** |
| **triply-charged** | | | |
| **GIIVR** | **279.2** | **500.4** | **2y4** |
| **(SEQ ID NO: 201)** | **279.2** | **274.2** | **2y2** |
| | | | |
| **doubly-charged** | | | |
| **GLPLDVIQNSFDISR** | **837.5** | **1079.6** | **2y9** |
| **(SEQ ID NO: 202)** | **837.5** | **966.5** | **2y8** |
| | **837.5** | **752.6** | **2y13(2)** |
| **doubly-charged** | | | |
| **SEMTIFAPGSR** | **598.3** | **747.4** | **2y7** |
| **(SEQ ID NO: 203)** | **598.3** | **848.5** | **2y8** |
| | **598.3** | **634.3** | **2y6** |
| **doubly-charged** | | | |

**Table 19**

| **Walnut Proteins** | **Q1** | **Q3** | **Fragment Ion** |
|---|---|---|---|
| **QQNLNHCQYYLR** | **813.4** | **1142.5** | **2y8** |
| **(SEQ ID NO: 204)** | | | |
| | | | |
| **doubly-charged** | | | |
| **QCCQQLSQMDEQCQCEGLR** | **805.3** | **900.3** | **3y7** |
| **(SEQ ID NO: 205)** | **805.3** | **623.3** | **3y5** |
| | **805.3** | **345.2** | **3y3** |
| **triply-charged** | | | |
| **GEEMEEMVQSAR** | **698.3** | **820.4** | **2y7** |
| **(SEQ ID NO: 206)** | **698.3** | **949.4** | **2y8** |
| | **698.3** | **461.3** | **2y4** |
| **doubly-charged** | | | |
| **DLPNECGISSQR** | **682.8** | **1136.5** | **2y10** |
| **(SEQ ID NO: 207)** | **682.8** | **796.3** | **2y7** |
| | **682.8** | **925.4** | **2/y8** |
| **doubly-charged** | **682.8** | **1039.4** | **2/y9** |
| | **682.8** | **568.7** | **2/y10(2)** |
| **LDALEPTNR** | **514.8** | **729.4** | **2/y6** |
| **(SEQ ID NO: 208)** | **514.8** | **487.3** | **2/y4** |
| | **514.8** | **616.3** | **2/y5** |
| **doubly-charged** | | | |
| **GLGNNVFSGFDADFLADAFNVDTETAR** | **955.1** | **692.3** | **3/y6** |
| **(SEQ ID NO: 209)** | **955.1** | **905.4** | **3/y8** |
| | **955.1** | **577.3** | **3/y5** |
| **triply-charged** | | | |
| **WLQLSAER** | **501.8** | **703.4** | **2/y6** |
| **(SEQ ID NO: 210)** | **501.8** | **816.5** | **2/y7** |
| | **501.8** | **575.3** | **2/y5** |
| **doubly-charged** | | | |
| **ALPEEVLATAFQIPR** | **792.4** | **903.5** | **2/y8** |
| **(SEQ ID NO: 211)** | **792.4** | **1016.6** | **2/y9** |
| | | | |
| **doubly-charged** | | | |
| **ALPEEVLATAFQIPR** | **528.6** | **1016.6** | **3/y9** |
| **(SEQ ID NO: 212)** | | | |
| | | | |
| **triply-charged** | | | |

Two or more of the above allergens can be detected and/or quantified by detecting, via LC-MS/MS, one or more peptides specific for each allergen, such as those discussed above, where for each peptide multiple MRM transitions, such as those discussed above, can be monitored. In one embodiment, for example, egg proteins can be detected in a sample by monitoring multiple MRM transitions for two peptides specific to ovalbumin (e.g., monitoring the MRM transitions of 930/1116,930/888, and 930/1017 for the peptide having the amino acid sequence of SEQ. ID NO. 1 and monitoring the MRM transitions 390/667,390/504, and 390/433 for the peptide having the amino acid sequence of SEQ. ID NO. 7) and one peptide specific to lysozyme (e.g., monitoring the MRM transitions of 437/452,437/680, and 437/737 for the peptide having the amino acid sequence of SEQ. ID NO. 8). Milk proteins can be detected in a sample by monitoring multiple MRM transitions for two peptides specific to casein isoform S1 (e.g., monitoring the MRM transitions of 693/920,693/991, and 693/1090 for the peptide having the amino acid sequence of SEQ. ID NO. 12 and monitoring the MRM transitions of 880/1324, 880/436, 587/758, 587/871, and 587/790 for the peptide having the amino acid sequence of SEQ. ID NO. 16), one peptide specific to casein isoform S2 (e.g., monitoring the MRM transitions of 598/911 and 598/456 for the peptide having the amino acid sequence of SEQ. ID NO. 20), and one specific to lactoglobulin (e.g. monitoring the MRM transitions of 623/1047, 623/918, 623/819 for the peptide having the amino acid sequence of SEQ. ID NO. 27). Any combination of any of the allergens disclosed in Tables 1 to 19 can be detected and/or quantified using similar procedures.

According to some embodiments, a kit for use in the mass spectrometric testing of a sample for ovalbumin, is provided. The kit can include one or more isolated peptides specific to chicken ovalbumin. The kit includes one or more isolated ovalbumin-specific peptides having the amino acid sequence of SEQ ID NO: 1, or SEQ ID NO: 3. In some embodiments, the peptides can be isotopically labeled (e.g., using 15N, 13C).

The kit includes at least one proteolytic enzyme for fragmenting ovalbumin into a plurality of peptides.

Various processes and reagents can be effective to prepare the sample for mass spectrometric analysis and/or fragment the allergens into a plurality of peptides. For example, in one exemplary embodiment, the proteolytic enzyme can be trypsin that can lyse the ovalbumin into a plurality of peptides. In some embodiments, the kit can include an LC column on which a proteolytic enzyme, such as trypsin, is immobilized. The kit can also comprise digestion components, including buffers enzymes, alkylating agents, reducing agents, and optionally, other reagents and/or components. In some embodiments, the kit can comprise, for example, a homogeneous assay such that the user need only add a sample.

In some embodiments, the kit can comprise calibration or normalization reagents or standards. For example, the kit can comprise at least one peptide specific to ovalbumin for calibrating the quantitation of the ovalbumin-specific peptides. By way of non-limiting example, the kit can contain solutions for ovalbumin-specific peptides at known concentrations such that a calibration curve can be constructed. In some embodiments, the kit can contain ovalbumin for calibrating the quantitation of the ovalbumin-specific peptides or ovalbumin itself. For example, the kit can include a known amount of ovalbumin. Alternatively or in addition, calibration can be performed by spiking the sample with at least one isotopically-enriched peptide having the same amino acid sequence as that a peptide of interest. Accordingly, the kit includes one or more isotopically-enriched peptides corresponding to SEQ ID NOS. 1 or 3.

According to some embodiments, different transitions can be used to measure and benchmark assay results, depending on various factors. Accordingly, the kit can comprise different transition values and/or suggested settings, useful for enabling comparative measurements between a sample and one or more control reagents. The kit can include information relating to Q1 and Q3 transition values for ovalbumin-specific peptides. For example, in one embodiment, the kit can comprise the isolated peptides of SEQ ID NOS: 1 or 3 identified herein, and further can comprise instructions for quantifying the at least one of the peptides using a mass spectrometer. Information pertaining to instrument settings that can or should be used to perform an assay can also be included in the kit. Information pertaining to sample preparation, operating conditions, volumetric amounts, temperature settings, and the like, can be included with the kit.

According to some embodiments, different transitions can be used to measure and benchmark assay results, depending on various factors. Accordingly, the kit can comprise different transition values and/or suggested settings, useful to make comparative measurements between a sample and one or more control reagents. The kit can include instructions to measure specific pairs of transition values, for example, the Q1/Q3 transition pair, or the values of one or more different transition pairs.

The kit can be packaged in a hermetically sealed container containing one or more reagent vessels and appropriate instructions. An electronic medium can also be contained within the kit and can store and/or provide electronic information pertaining to one or more assays, measurement values, transition pairs, operating instructions, software for carrying out operations, a combination thereof, or the like.

According to some aspects, software is provided which can control the processes and/or perform the calculations described herein. For example, the software can provide instructions to a mass spectrometer to monitor one or more specific precursor-product ion pair transitions.

The software can include modules for generating calibration data, e.g., based on mass spectrometric analysis of calibrations standards provided with a kit, and modules for receiving and analyzing mass spectrometric data (e.g., LC-MS/MS data) to identify one or more of the above peptides and MRM transitions. Upon identification of one or more peptides specific to one of the above allergens, the software can utilize the calibration data to quantify those peptides and the associated allergen.

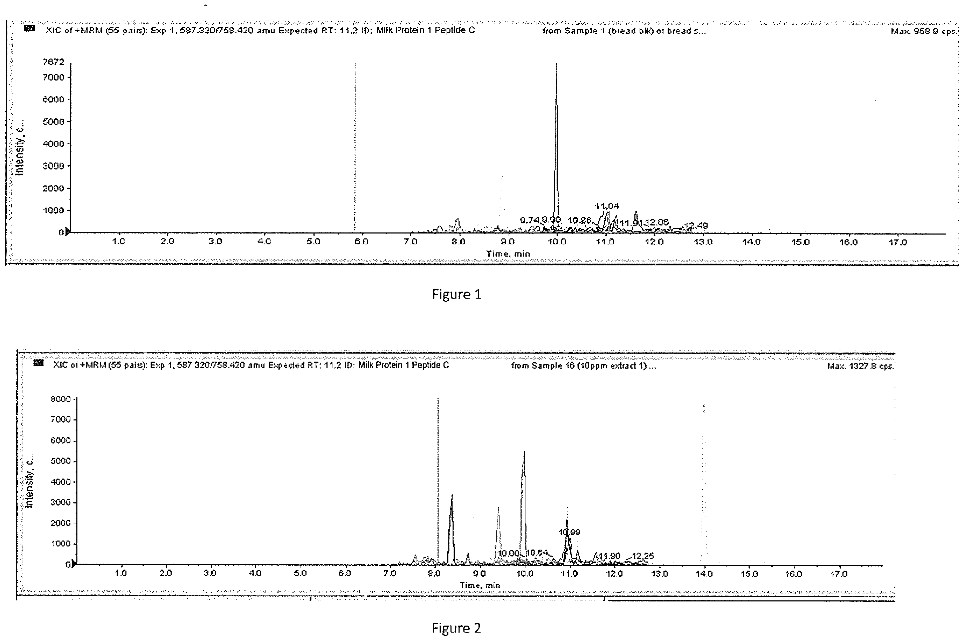

FIGURE 1 shows an exemplary chromatogram resulting from an exemplary "blank" sample of bread.
FIGURE 2 shows an exemplary chromatogram resulting from a bread sample spiked with milk, and containing milk peptides.

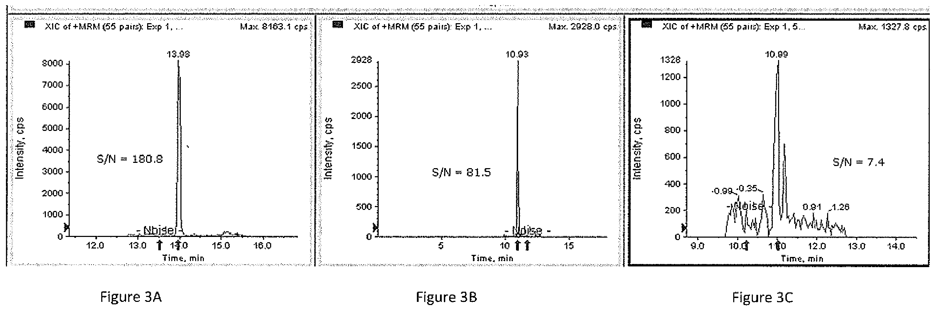

FIGURES. 3A, 3B, 3C depict the signals obtained for three specific milk peptides.
FIGURE 4 shows a mass spectra of a sample containing mustard.
FIGURE 5 show a calibration line for a peptide of SEQ ID NO. 20.
FIGURE 6 show a calibration line for a peptide of SEQ ID NO. 16.
FIGURE 7 show a calibration line for a peptide of SEQ ID NO. 12.
FIGURE 8 show a calibration line for a peptide of SEQ ID NO. 27.
FIGURE 9 show a calibration line for a peptide of SEQ ID NO. 7.

### EXAMPLES

The applicants' teachings can be even more fully understood with reference to the examples and resulting data that follow. Other embodiments of the applicants' teachings will be apparent to those skilled in the art from consideration of the present specification and practice of the present teachings disclosed herein. It is intended that these examples be considered as exemplary only.

### Example 1

### Preparation of solutions

Extraction Bufter-Tris (2-Amino-2-hydroxymethyl-propane-1,3-diol, 3.03 g) and Urea (60 g) were dissolved into water (480 mL) make a 50mM Tris buffer/2 M Urea extraction solvent. The resulting solution had a pH of approximately 10.

Digestion Bufter-Ammonium bicarbonate (3.16 g) was dissolved in water (400 mL). The resulting solution had a pH of approximately 7.8.

Enzyme solution - Trypsin (1g) was dissolved in digestion buffer (20 mL) to make a stock solution at a concentration of 50 mg/mL. The stock solution (100 µL) was further diluted into 10 mL of digestion buffer to make a final trypsin solution of 500µg/mL solution. All enzyme solution were frozen at -20°C (or lower) for storage and thawed prior to use.

lodoacetamide solution - lodoacetamide (0.925 g) was added to 10 mL of water and sonicated to dissolve the solid. The 0.5M iodoacetamide solution was prepared fresh prior to use and discarded after use.

Preparation of Mixed Allergen spike solution - 20.0 mg (0.0200g) of each protein (Egg lysozyme, Egg albumin, Milk α-casein, and Milk β-lactoglobulin) is dissolved in 10.0 mL of extraction buffer in a 15mL polypropylene centrifuge tube to make a combined spiking solution containing 2000 µg/mL of each allergen. The resulting Mixed Allergen Spiking solution is stable for 1 month when stored in a refrigerator. Longer term stability has not been determined.

### Sample Preparation

As discussed above, the samples can be derived from a variety of sources. In this example, the sample was obtained from dry baked foodstuffs. Finely powdered sample (5.0 g) was mixed in a centrifuge tube (50 mL) with 30 mL of extraction buffer and the mixture was broken up by vigorous shaking and agitated further using a roller mixer (60 minutes). The centrifuge tubes were spun down for 5 minutes at 3500 rpm and 15°C. 200 µL of 1M DTT solution is added to 6 mL of the supernatant. The samples were mixed and heated for 60 minutes at 37°C. The samples were then cooled to room temperature and 2.0 mL of freshly prepared 0.5M iodoacetamide solution in water was added. The samples were shaken to mix and stored protected from light (30 minutes at room temperature). To each sample, 6 mL of digestion buffer (0.1M ammonium bicarbonate) was added, followed by 400 µL of trypsin enzyme solution (200 µg total enzyme per sample). The samples were incubated overnight at 37°C. The samples were centrifuged (5 minutes, 10°C at 3000 rpm) and filtered through 0.45µ RC filter. The filtrate was acidified with 0.5 mL of formic acid.

Strata™ X SPE cartridges were conditioned with 6mL of acetonitrile containing 0.1% formic acid followed with 6mL of water containing 0.5% TFA (trifluoroacetic acid). Supernatant from the centrifuged samples were loaded onto the SPE cartridges at a rate of 1-2 drops per second. The SPE cartridges were washed with 3mL of 0.5% TFA in water. Allergen peptides were eluted with 6mL of acetonitrile and evaporated to dryness using a centrifugal evaporator.

The sample residue was reconstituted by adding 300µL of a water/acetonitrile solution (95% water with 5% acetonitrile and 0.5% formic acid). The samples were vortexed for 20-30 seconds and sonicated for 30 minutes to complete dissolution of material. The contents were transferred to a 1.5mL Eppendorf tube and centrifuged (5 minutes at 13000 rpm). The supernatant was removed and transferred to a 300µL polypropylene HPLC vial with press fit cap for HPLC analysis.

Chromatography was performed using a Shimadzu Prominence LC system using a Phenomenex Analytical Column, 4 µm, Synergi Hydro-RP 80A Column 150 x 2.1 mm using a mobile phase solutions of 1.00ml of formic acid in 999 ml of water and 1.00ml of formic acid in 999ml of acetonitrile. Flow rate was 0.300 ml/min at a column oven temperature of 30C.

Samples were analyzed on an Applied Biosystems 4000QTRAP® LC/MS/MS system using a TurbolonSpray® ion source. The compounds were analyzed using scheduled MRM (sMRM) with a MRM detection window of 90 s and a target scan time of 0.40 s. Q1 was set to low and Q3 was set to unit resolution to obtain maximum signal response.

### Example 2

Another method to prepare the samples is described in which in this example. A NANOSEP 10k OMEGA (100/pk) and a Phenex RC membrane, 0.45 µm, 26 mm syringe filters. In addition, the following HPLC Solvents and Consumables were utilized: Water, Methanol (MeOH), Acetonitrile (CAN), 50 mL polypropylene centrifuge tubes, 15 mL polypropylene centrifuge tubes, Disposable glass culture tubes (13 x 100 mm), Polypropylene microcentrifuge tubes (1.5 mL), Conical polypropylene HPLC vials and caps (300 µL), Disposable syringe, 10 mL capacity, luer end. Furthermore, the following equipment was necessary for extractions and sample processing: Ultracentrifuge for 50 mL tubes, Microcentrifuge for 1.5 mL tubes, SPE manifold for sample processing (with vacuum pump), Forced air incubator (set to 37°C), roller shaker, Centrifugal evaporator/concentrator.

### Preparation of solutions

Extraction Bufter-Tris (2-amino-2-hydroxymethyl-propance-1,3-diol, 3.03 g) and urea (60 g) was dissolved into water (480 ml) and stirred until complete dissolution to make a 50mM Tris buffer/2 M Urea extraction solvent.

Digestion Buffer-Ammonium bicarbonate (3.16 g) was dissolved in water (378 mL). To this was added 2 ml of 1M calcium chloride and 20 ml of acetonitrile. The pH was approximately 7.8.

Enzyme solution - Trypsin (1g) was dissolved in digestion buffer (20 mL) to make a stock solution at a concentration of 50 mg/mL. The stock solution (100 µL) was further diluted into 10 mL of digestion buffer to make a final trypsin solution of 500µg/mL solution. All enzyme solution were frozen at -20°C (or lower) for storage and thawed prior to use.

Preparation of Individual Allergen Stock Solution - -A 10 mg/ml stock solution was prepared by dissolving 30.0 mg each of egg albumin, milk casein and lactoglobulin) in 3.0 ml of extraction buffer in a 5 ml Eppendorf tube. The resulting allergen stock solutions were stable for three months when stored at -20C or lower, though longer term stability has not been determined.

Preparation of Mixed Allergen spike solution - 1.0 ml of each allergen stock solution (10 mg/ml) (egg albumin, mile casein and milk lactoglobulin) is transferred into a 5 ml Eppendorf tube and to this 2.0 ml of the extraction buffer was added and mixed. The mixed allergen stock solution contained 2 mg/ml of each allergen and is stable for one month when stored at -20C or lower though longer term stability has not been determined.

Preparation of Mixed Allergen Spike Solution - 100 µL of the mixed allergen stock solution (2 mg/ml) was added to a 2 mL eppendorf tube containing 900 µL of the extraction buffer. The resulting solution contained 200 µg/ml of each allergen protein.

### Sample Preparation

1.00 g of a finely powdered sample was weighed into a 15 ml centrifuge tube. Serial addition calibration samples from each individual sample were prepared from each individual samples following table 20.

**Table 20**

| **Calibration Standard** | **Sample Weight (g)** | **Spike Solution Used** | **Spike Volume (µL)** | **Extraction Buffer Volume (mL)** |
|---|---|---|---|---|
| **Sample** | **1.00** | **Mixed Allergens** | **0** | **6.00** |
| **Sample + 4 ppm** | **1.00** | **Mixed Allergens** | **20** | **5.98** |
| **Sample + 10 ppm** | **1.00** | **Mixed Allergens** | **50** | **5.95** |
| **Sample + 40 ppm** | **1.00** | **Mixed Allergens** | **200** | **5.80** |
| **Sample + 100 ppm** | **1.00** | **Mixed Allergens** | **500** | **5.50** |

Each mixture was shaken vigorously to break the mixture down and then agitated further for 60 minutes using a roller mixer. The centrifugal tubes were spun down for 20 minutes at 3500 rpm and 15C. 500 µL of each supernatant was added to a 2ml eppendorf tube and 50 µL of TCEP solution and vortex mixed in incubated for 60 minutes at 60C. The tubes were then allowed to cool to room temperature at 25 µL of MMTS were added, vortexed again to mix and incubated for 10 minutes. 500 µL of digestion buffer were added to each sample followed by 20 µL of trypsin enzyme solution (containing 20 µf total enzyme per sample). Solution was mixed thoroughly by vortex. The samples were then incubated overnight at 37C. The next day 30 µL of formic acid was added to each sample and mixed well. The samples were then centrifuged for 5 minutes at 13000 rpm. 500 µL of the supernatant were taken into a 10K-MwCO filter and centrifuged for 20 minutes at 13000 rpm. 200 µL of the filtrate were transferred into an appropriate polypropylene HPLC autosampler vial for LC/MS/MS analysis with the remainder being stored at -20C for further analysis if necessary.

Chromatography was performed using a Shimadzu Prominence LC system using a Phenomenex Analytical Column, 4 µm, Synergi Hydro-RP 80A Column 150 x 2.1 mm using a mobile phase solutions of 1.00ml of formic acid in 999 ml of water and 1.00ml of formic acid to 999 ml of acetonitrile. The system also consisted of System controller (CBM-20A), 2 Isocratic pumps LC-20AD (with semi-micro 50 µL mixer), an Autosampler SIL-20AC and a Column oven CTO-20AC. In addition, a Guard Column (Phenomenex trap cartridge, 4 µm, Synergi Hydro-RP 80A Mercury, 20 x 2.0 mm), a cartridge holder (Phenomenex MercuryMS 20mm Cartridge Holder) were utilizes and a needle rinse of 95:5 (v:v) acetonitrile: water + 0.05% TFA was utilized with a Flow Rate: 0.300 mL/min, Equilibration time: 0.5 min, Column oven temperature: 30°C, and an Injection volume: 30 µL. The Autosampler needle rinse sequence included Rinsing Volume: 1000 µL, Needle stroke: 52 mm, Rinsing speed: 35 µL/sec, Sampling speed: 2.0 µL/sec, Rinse dip time: 5 sec, Rinse mode: Before and after aspiration.

Samples were analyzed on an Applied Biosystems 4000QTRAP® LC/MS/MS system using a TurbolonSpray® ion source with positive polarity. The compounds were analyzed using scheduled MRM (sMRM) with a MRM detection window of 90 s and a target scan time of 0.40 s. Q1 was set to low and Q3 was set to unit resolution to obtain maximum signal response. Other parameters utilizes included Source/Gas Parameters IS: 5500, CUR: 30 psi, TEM: 500°C, GS1: 35 psi, GS2: 46 psi, Ihe: On, and CAD: High.

The Compound MRM Parameters and Retention Times included DP: variable, EP: 10, CXP: variable, Dwell time: variable, Q1 Resolution: LOW, Q3 and Resolution: UNIT

Calibration curves were derived from absolute peak areas using a least squares regression of the area versus the nominal concentrations of the serial spiked samples. Deviations from the regression line are calculated by using the regression equation to back calculate the concentration at each spiking level. Concentrations of target peptides are obtained from the regression curves calculated from the apparent concentration of the unspiked samples. The linear ranges for each of the egg albumin, egg lysozyme, milk casein and lactoglobulin ranges between 10 and 100 µg/g

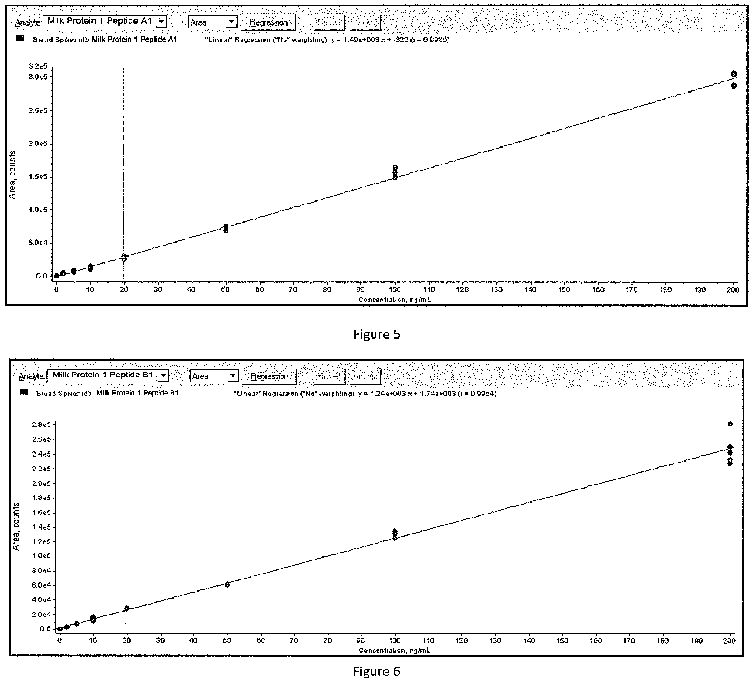

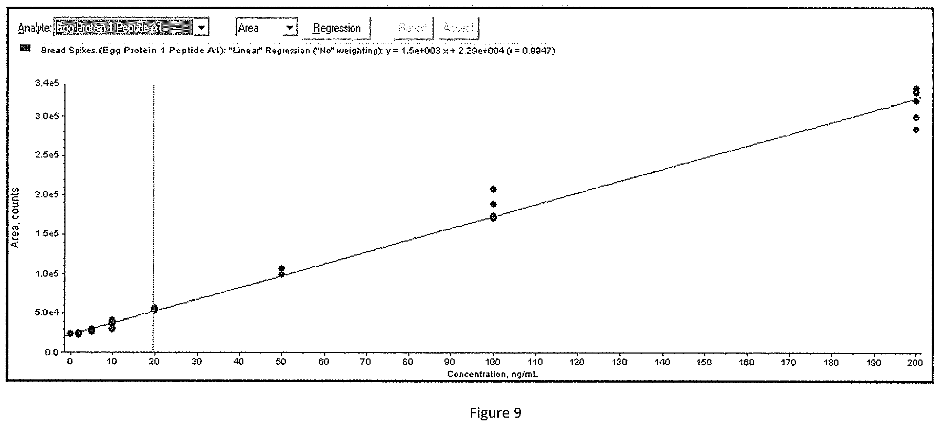

Each sample has its own linear range for quantitation. Calibration standards in the full calibration curve should be excluded from the regression to meet the applicable range. The calibration curves are depicted in Figures 5 to 9.

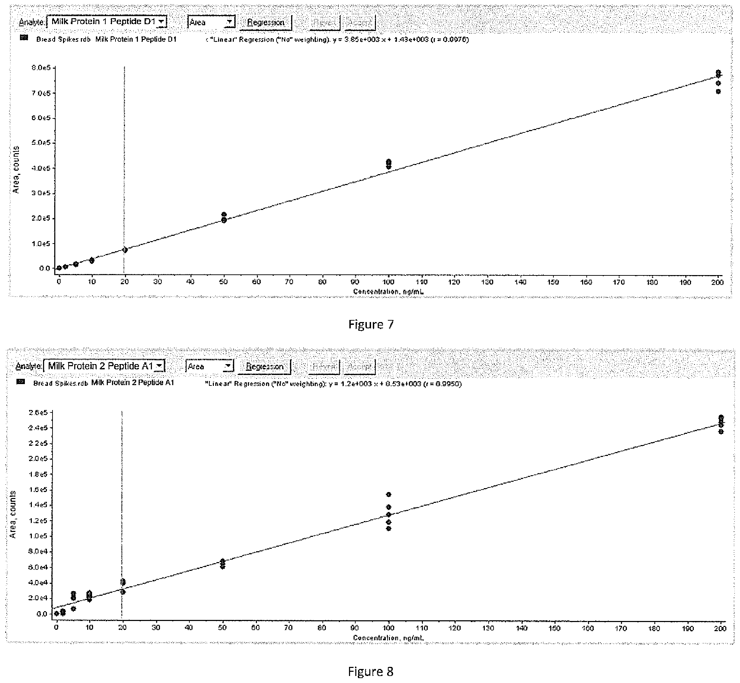

Figure 5 depicts the calibration line for a casein peptide of SEQ ID NO. 20 when 200 ppm spiked into bread. Figure 6 depicts the calibration line for a casein peptide of SEQ ID NO. 16 when 200 ppm is spiked into bread. Figure 7 depicts the calibration line for a casein peptide of SEQ ID NO. 12 when 200 ppm spiked into bread. Figure 8 depcits the calibration line for a bovine peptide of SEQ ID NO. 27 when 200 ppm is spiked into bread. Figure 8 depicts the calibration line for an ovalbumin peptide of SEQ ID NO. 7 when 200 ppm is spiked into bread.

### Example 3

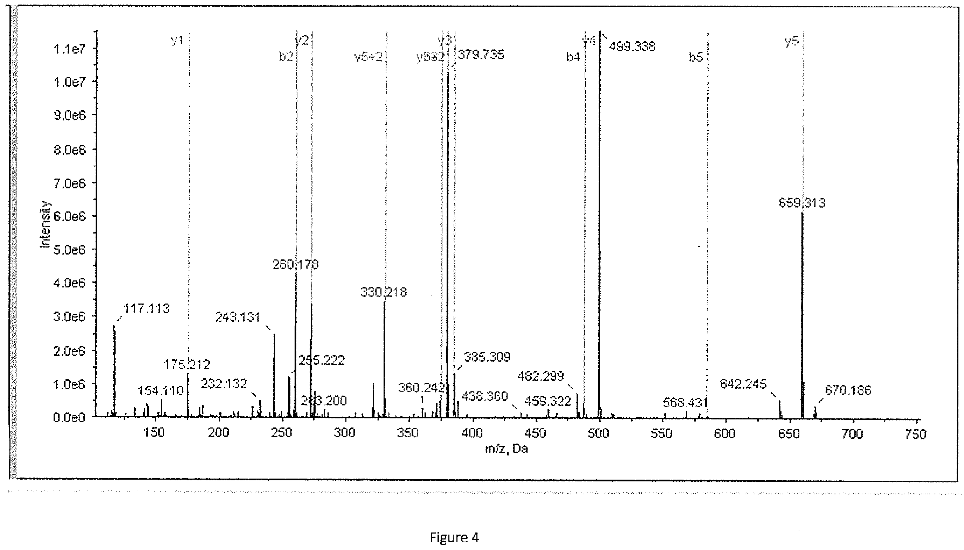

A package of mustard was combined with 100mM of Tris and 2M Urea. The pH was adjusted to a pH of 9 and 9.5 using ammonium hydroxide to extract relevant proteins. The sample was subjected to similar preparation and analysis technique as described in Examples 1 or 2 with an additional process in which iodoacetamide was added to block the cysteine. The peptide sequence SEQ ID NO. 103 that had its cysteine modified with iodoacetamide was isolated and analyzed. Figure 4 depicts a mass spectra of this sample. As is evident, the mass spectra peaks are identified at 380/499 corresponding to fragment ion at 2/y4, at 380/659 corresponding to a fragment ion at 2/y5, at 380/584 correspond to a fragment ion at 2/b5 (minor peak) and at 380/487, corresponding to a fragment ion at 2/b4.

The quantification of peptides and/or allergens of interest can utilize various calibration techniques. For example, a calibration standard containing a known amount of a selected peptide can be added to the sample prior to mass spectrometric analysis to generate a calibration curve that allows for the quantitation of samples that are subsequently run through an LC-MS/MS. In some embodiments, a known amount of the allergen(s) of interest can be added to the sample prior to preparing the sample for mass spectrometric analysis. By way of example, a known amount of one or more of ovalbumin, lysozyme, casein, lactoglobulin, high or low glutins, wheat, rye, oats, barley, mustard, sesame and various nuts including macadamia, pistachio, brazil, walnuts, peanuts and hazelnuts can be added to the sample prior to digesting the sample with a proteolytic enzyme. By spiking the sample with a known concentration of the allergen(s) of interest, the quantitation of the allergen(s) or their specific peptides can be calibrated. In some embodiments, multiple samples spiked with various known amounts of the allergen(s) of interest can be iteratively tested to generate a calibration curve, for example, using a serial dilution or standard addition method. Calibration curves of the data resulting from the mass spectrometric analysis can be used to calculate, for example, the apparent concentration of peptides and/or allergen(s) in the "unspiked" samples. Various calibration curves and further detail regarding one embodiment of a calibration protocol are further shown in Figures 5 to 9 depcit calibration curves for various peptides.

In some embodiments, the quantity of the peptide can be determined, for example, by Isotope Dilution Mass Spectrometry, wherein the prepared sample is spiked with an isotopically-enriched form of a peptide of interest. In one exemplary embodiment, the calibration standard can be "spiked" into the sample using various concentrations for different runs (e.g., serial dilution). Calibration curves of the data resulting from the mass spectrometric analysis can be used to calculate, for example, the apparent concentration of "unspiked" samples.

One skilled in the art will appreciate further features and advantages of methods and systems in accord with applicants' teachings based on the above-described embodiments. Accordingly, the applicants' disclosure is not to be limited by what has been particularly shown and described, except as indicated by the appended claims.

## Claims

1. A method of detecting ovalbumin in a sample, said method comprising:
adding a proteolytic enzyme to the sample to lyse at least a portion of any ovalbumin present in the sample, into a plurality of peptides; and
utilizing liquid chromatography tandem mass spectrometry (LC-MS/MS) to analyze said plurality of peptides to determine whether ovalbumin is present by monitoring at least one precursor-product ion pair transition with specified m/z value associated with a specific amino acid sequence selected from the group consisting of:
i) SEQ ID NO: 1, m/z value of about 391/504, 391/667, or 391/433;
ii) SEQ ID NO: 3, m/z value of about 605/419, 605/621, or 605/749;
iii) SEQ ID NO: 5, m/z value of about 791/1052, 791/951, or 791/1239; and
iv) SEQ ID NO: 6, m/z value of about 844/666, 844/1332, or 844/1121.

2. The method of claim 1, wherein the proteolytic enzyme comprises trypsin.

3. The method of claim 1, wherein utilizing LC-MS/MS to analyze said plurality of peptides comprises quantifying an amount of one of said plurality of peptides having SEQ ID NO 1, 3, 5 or 6 by utilizing at least one isotopically-enriched peptide having SEQ ID NO 1, 3, 5 or 6, and a standard comprising a known concentration of a peptide having SEQ ID NO 1, 3, 5 or 6 to calibrate the quantitation of said one of said plurality of peptides.

4. A kit for use in the mass spectrometric testing of a sample for ovalbumin, comprising:
at least one proteolytic enzyme for fragmenting ovalbumin into a plurality of peptides; and
at least one reagent for quantifying at least one of said plurality of peptides using a mass spectrometer, wherein said at least one of said plurality of peptides comprises a peptide having an amino acid sequence of SEQ ID NO: 1 or 3, wherein said at least one reagent comprises at least one isotopically-enriched peptide having an amino acid sequence of SEQ ID NO: 1 or 3.

5. The kit of claim 4, wherein said at least one reagent comprises a selected concentration of a peptide having an amino acid sequence of SEQ ID NO: 1 or 3.

6. The kit of claim 4, further comprising an alkylating agent, a reducing agent, instructions for quantifying ovalbumin using a mass spectrometer or information pertaining to transition pairs to be used as settings in a mass spectrometer.

## Patentansprüche

1. Verfahren zum Nachweis von Ovalbumin in einer Probe, wobei das Verfahren Folgendes umfasst:
Hinzugeben eines proteolytischen Enzyms zu der Probe, um mindestens einen Teil eines Ovalbumin, das in der Probe vorhanden ist, in eine Vielzahl von Peptiden zu lysieren; und
Anwenden der Flüssigchromatographie-Tandem-Massenspektrometrie (LC-MS/MS), um die Vielzahl von Peptiden zu analysieren, um zu bestimmen, ob Ovalbumin vorhanden ist, indem mindestens ein Vorläuferprodukt-Ionenpaarübergang mit einem bestimmten m/z-Wert überwacht wird, der einer spezifischen Aminosäuresequenz zugeordnet ist, die aus der Gruppe ausgewählt wird, die aus Folgendem besteht:
i) SEQ ID NO: 1, m/z-Wert von etwa 391/504, 391/667 oder 391/433;
ii) SEQ ID NO: 3, m/z-Wert von etwa 605/419, 605/621 oder 605/749;
iii) SEQ ID NO: 5, m/z-Wert von etwa 791/1052, 791/951 oder 791/1239 und
iv) SEQ ID NO: 6, m/z-Wert von etwa 844/666, 844/1332 oder 844/1121.

2. Verfahren nach Anspruch 1, wobei das proteolytische Enzym Trypsin umfasst.

3. Verfahren nach Anspruch 1, wobei die Verwendung von LC-MS/MS zur Analyse der Vielzahl von Peptiden das Quantifizieren einer Menge eines der Vielzahl von Peptiden mit der SEQ ID NO 1, 3, 5 oder 6 unter Verwendung von Folgendem umfasst: mindestens eines isotopisch angereicherten Peptids mit der SEQ ID NO 1, 3, 5 oder 6 und eines Standards, der eine bekannte Konzentration eines Peptids mit der SEQ ID NO 1, 3, 5 oder 6 umfasst, um die Quantifizierung des einen der Vielzahl von Peptiden zu kalibrieren.

4. Kit zur Verwendung bei der massenspektrometrischen Prüfung einer Probe für Ovalbumin, das Folgendes umfasst:
mindestens ein proteolytisches Enzym zum Fragmentieren von Ovalbumin in eine Vielzahl von Peptiden; und
mindestens ein Reagenz zum Quantifizieren von mindestens einer der Vielzahl von Peptiden unter Verwendung eines Massenspektrometers, wobei das mindestens eine der Vielzahl von Peptiden ein Peptid mit einer Aminosäuresequenz von SEQ ID NO: 1 oder 3 umfasst, wobei das mindestens eine Reagenz mindestens ein isotopisch angereichertes Peptid mit einer Aminosäuresequenz von SEQ ID NO: 1 oder 3 umfasst.

5. Kit nach Anspruch 4, wobei das mindestens eine Reagenz eine ausgewählte Konzentration eines Peptids mit einer Aminosäuresequenz von SEQ ID NO: 1 oder 3 umfasst.

6. Kit nach Anspruch 4, das ferner Folgendes umfasst: ein Alkylierungsmittel, ein Reduktionsmittel, Anweisungen zur Quantifizierung von Ovalbumin mit einem Massenspektrometer oder Informationen über Übergangspaare, die als Einstellungen in einem Massenspektrometer zu verwenden sind.

## Revendications

1. Procédé de détection d'ovalbumine dans un échantillon, ledit procédé comprenant :
l'ajout à l'échantillon d'une enzyme protéolytique pour lyser au moins une partie d'une albumine éventuellement présente dans l'échantillon en une pluralité de peptides ; et
l'utilisation d'une chromatographie liquide couplée à une spectrométrie de masse (CL-SM/SM) pour analyser ladite pluralité de peptides afin de déterminer si une ovalbumine est présente en surveillant au moins une transition de paire d'ions précurseurs/produits de valeur m/z spécifiée associée à une séquence d'acides aminés choisie dans le groupe constitué par :
i) SEQ ID NO : 1, valeur m/z d'environ 391/504, 391/667 ou 391/433 ;
ii) SEQ ID NO : 3, valeur m/z d'environ 605/419, 605/621 ou 605/749 ;
iii) SEQ ID NO : 5, valeur m/z d'environ 791/1052, 791/951 ou 791/1239 ; et
iv) SEQ ID NO : 6, valeur m/z d'environ 844/666, 844/1332 ou 844/1121.

2. Procédé de la revendication 1, dans lequel l'enzyme protéolytique comprend de la trypsine.

3. Procédé de la revendication 1, dans lequel l'utilisation d'une CL-SM/SM pour analyser ladite pluralité de peptides comprend la quantification d'une quantité de l'un de ladite pluralité de peptides ayant SEQ ID NO : 1, 3, 5, ou 6 par l'utilisation d'au moins un peptide isotopiquement enrichi ayant SEQ ID NO : 1, 3, 5 ou 6 et d'un étalon comprenant une concentration connue d'un peptide ayant SEQ ID NO : 1, 3, 5 ou 6 pour étalonner la quantification dudit un de ladite pluralité de peptides.

4. Nécessaire à utiliser en spectrométrie de masse pour le dosage d'ovalbumine dans un échantillon, comprenant :
au moins une enzyme protéolytique destinée à fragmenter l'ovalbumine en une pluralité de peptides ; et
au moins un réactif destiné à quantifier au moins l'un de ladite pluralité de peptides à l'aide d'un spectromètre de masse, ledit au moins un de ladite pluralité de peptides comprenant un peptide ayant une séquence d'acides aminés de SEQ ID NO : 1 ou 3, ledit au moins un réactif comprenant au moins un peptide isotopiquement enrichi ayant une séquence d'acides aminés de SEQ ID NO : 1 ou 3.

5. Nécessaire de la revendication 4, dans lequel ledit au moins un réactif comprend une concentration choisie d'un peptide ayant une séquence d'acides aminés de SEQ ID NO : 1 ou 3.

6. Nécessaire de la revendication 4, comprenant en outre un agent alcalinisant, un agent réducteur, des instructions pour quantifier l'ovalbumine à l'aide d'un spectromètre de masse ou des informations concernant des paires de transition à utiliser comme réglages dans un spectromètre de masse.


### SEQUENCE LISTING

<110> Lock, Stephen Purkayastha, Subhasish Williamson, Brian Dong, Keling
<120> Systems and Methods for the Detection of Allergens
<130> 110323-589
<141> 2012-xx-xx
<150> 61/530612
   <151> 2011-09-02
<160> 212
<170> PatentIn version 3.5
<210> 1
   <211> 6
   <212> PRT
   <213> Gallus gallus
<400> 1
   Leu Tyr Ala Glu Glu Arg
   1               5
<210> 2
   <211> 12
   <212> PRT
   <213> Gallus gallus
<400> 2
   Tyr Pro Ile Leu Pro Glu Tyr Leu Gln Cys Val Lys
   1               5                   10
<210> 3
   <211> 10
   <212> PRT
   <213> Gallus gallus
<400> 3
   Asp Glu Asp Thr Gln Ala Met Pro Phe Arg
   1               5                   10
<210> 4
   <211> 12
   <212> PRT
   <213> Gallus gallus
<400> 4
   His Ile Ala Thr Asn Ala Val Leu Phe Phe Gly Arg
   1 5 10
<210> 5
   <211> 13
   <212> PRT
   <213> Gallus gallus
<400> 5
   Leu Thr Glu Trp Thr Ser Ser Asn Val Met Glu Glu Arg
   1 5 10
<210> 6
   <211> 16
   <212> PRT
   <213> Gallus gallus
<400> 6
   Gly Gly Leu Glu Pro Ile Asn Phe Gln Thr Ala Ala Asp Gln Ala Arg
   1 5 10 15
<210> 7
   <211> 16
   <212> PRT
   <213> Gallus gallus
<400> 7
   Glu Leu Ile Asn Ser Trp Val Glu Ser Gln Thr Asn Gly Ile Ile Arg
   1 5 10 15
<210> 8
   <211> 7
   <212> PRT
   <213> Gallus gallus
<400> 8
   His Gly Leu Asp Asn Tyr Arg
   1 5
<210> 9
   <211> 7
   <212> PRT
   <213> Gallus gallus
<400> 9
   Trp Trp Cys Asn Asp Gly Arg
   1 5
<210> 10
   <211> 12
   <212> PRT
   <213> Gallus gallus
<400> 10
   Phe Glu Ser Asn Phe Asn Thr Gln Ala Thr Asn Arg
   1 5 10
<210> 11
   <211> 16
   <212> PRT
   <213> Gallus gallus
<400> 11
   Asn Thr Asp Gly Ser Thr Asp Tyr Gly Ile Leu Gln Ile Asn Ser Arg
   1 5 10 15
<210> 12
   <211> 12
   <212> PRT
   <213> Bos taurus
<400> 12
   Phe Phe Val Ala Pro Phe Pro Glu Val Phe Gly Lys
   1 5 10
<210> 13
   <211> 16
   <212> PRT
   <213> Bos taurus
<400> 13
   Leu Leu Ile Leu Thr Cys Leu Val Ala Val Ala Leu Ala Arg Pro Lys
   1 5 10 15
<210> 14
   <211> 7
   <212> PRT
   <213> Bos taurus
<400> 14
   Glu Asp Val Pro Ser Glu Arg
   1 5
<210> 15
   <211> 10
   <212> PRT
   <213> Bos taurus
<400> 15
   Tyr Leu Gly Tyr Leu Glu Gln Leu Leu Arg
   1 5 10
<210> 16
   <211> 15
   <212> PRT
   <213> Bos taurus
<400> 16
   His Gln Gly Leu Pro Gln Glu Val Leu Asn Glu Asn Leu Leu Arg
   1               5                   10                  15
<210> 17
   <211> 6
   <212> PRT
   <213> Bos taurus
<400> 17
   Val Asn Glu Leu Ser Lys
   1               5
<210> 18
   <211> 7
   <212> PRT
   <213> Bos taurus
<400> 18
   Glu Asp Val Pro Ser Glu Arg
   1               5
<210> 19
   <211> 6
   <212> PRT
   <213> Bos taurus
<400> 19
   Val Ile Pro Tyr Val Arg
   1               5
<210> 20
   <211> 11
   <212> PRT
   <213> Bos taurus
<400> 20
   Asn Ala Val Pro Ile Thr Pro Thr Leu Asn Arg
   1               5                   10
<210> 21
   <211> 8
   <212> PRT
   <213> Bos taurus
<400> 21
   Asn Met Ala Ile Asn Pro Ser Lys
   1               5
<210> 22
   <211> 8
   <212> PRT
   <213> Bos taurus
<400> 22
   Phe Ala Leu Pro Gln Tyr Leu Lys
   1 5
<210> 23
   <211> 12
   <212> PRT
   <213> Bos taurus
<400> 23
   Thr Val Asp Met Glu Ser Thr Glu Val Phe Thr Lys
   1 5 10
<210> 24
   <211> 9
   <212> PRT
   <213> Bos taurus
<400> 24
   Val Leu Val Leu Asp Thr Asp Tyr Lys
   1 5
<210> 25
   <211> 8
   <212> PRT
   <213> Bos taurus
<400> 25
   Ile Asp Ala Leu Asn Glu Asn Lys
   1 5
<210> 26
   <211> 20
   <212> PRT
   <213> Bos taurus
<400> 26
   Val Tyr Val Glu Glu Leu Lys Pro Thr Pro Glu Gly Asp Leu Glu Ile
   1 5 10 15
   Leu Leu Gln Lys
   20
<210> 27
   <211> 11
   <212> PRT
   <213> Bos taurus
<400> 27
   Thr Pro Glu Val Asp Asp Glu Ala Leu Glu Lys
   1 5 10
<210> 28
   <211> 9
   <212> PRT
   <213> Bos taurus
<400> 28
   Trp Glu Asn Gly Glu Cys Ala Gln Lys
   1 5
<210> 29
   <211> 7
   <212> PRT
   <213> Bos taurus
<400> 29
   Ala Leu Pro Met His Ile Arg
   1 5
<210> 30
   <211> 14
   <212> PRT
   <213> Unknown
<220>
   <223> Obtained from Hordeum vulgare
<400> 30
   Thr Leu Pro Met Met Cys Ser Val Asn Val Pro Leu Tyr Arg
   1 5 10
<210> 31
   <211> 8
   <212> PRT
   <213> Unknown
<220>
   <223> Obtained from Hordeum vulgare
<400> 31
   Gly Val Gly Pro Ser Val Gly Val
   1 5
<210> 32
   <211> 14
   <212> PRT
   <213> Unknown
<220>
   <223> Obtained from Hordeum vulgare
<400> 32
   Thr Leu Pro Thr Met Cys Ser Val Asn Val Pro Leu Tyr Arg
   1 5 10
<210> 33
   <211> 16
   <212> PRT
   <213> Unknown
<220>
   <223> Obtained from Hordeum vulgare
<400> 33
   Asn Thr Asp Gly Ser Thr Asp Tyr Gly Ile Leu Gln Ile Asn Ser Arg
   1 5 10 15
<210> 34
   <211> 7
   <212> PRT
   <213> Unknown
<220>
   <223> Obtained from genus Triticum species
<400> 34
   Ser Asp Cys Gln Val Met Arg
   1 5
<210> 35
   <211> 13
   <212> PRT
   <213> Unknown
<220>
   <223> Obtained from genus Triticum species
<400> 35
   Ala Pro Phe Ala Ser Ile Val Ala Ser Ile Gly Gly Gln
   1 5 10
<210> 36
   <211> 23
   <212> PRT
   <213> Unknown
<220>
   <223> Obtained from genus Triticum species
<400> 36
   Ser Leu Val Leu Gln Thr Leu Pro Thr Met Cys Asn Val Tyr Val Pro
   1 5 10 15
   Pro Tyr Cys Ser Thr Ile Arg
   20
<210> 37
   <211> 23
   <212> PRT
   <213> Unknown
<220>
   <223> Obtained from genus Triticum species
<400> 37
   Ser Leu Val Leu Gln Thr Leu Pro Thr Met Cys Asn Val Tyr Val Pro
   1 5 10 15
   Pro Tyr Cys Ser Thr Ile Arg
   20
<210> 38
   <211> 33
   <212> PRT
   <213> Unknown
<220>
   <223> Obtained from genus Triticum species
<400> 38
   Gln Pro Phe Pro Gln Gln Pro Gln Gln Pro Tyr Pro Gln Gln Pro Gln
   1 5 10 15
   Gln Pro Phe Pro Gln Thr Gln Gln Pro Gln Gln Pro Phe Pro Gln Ser
   20 25 30
   Lys
<210> 39
   <211> 7
   <212> PRT
   <213> Unknown
<220>
   <223> Obtained from genus Triticum species
<400> 39
   Val Ser Ile Ile Leu Pro Arg
   1 5
<210> 40
   <211> 15
   <212> PRT
   <213> Unknown
<220>
   <223> Obtained from genus Triticum species
<400> 40
   Gly Ile Ile Gln Pro Gln Gln Pro Ala Gln Leu Glu Gly Ile Arg
   1               5                   10                  15
<210> 41
   <211> 19
   <212> PRT
   <213> Unknown
<220>
   <223> Obtained from genus Triticum species
<400> 41
   Ala Gln Gly Leu Gly Ile Ile Gln Pro Gln Gln Pro Ala Gln Leu Glu
   1               5                   10                  15
   Gly Ile Arg
<210> 42
   <211> 23
   <212> PRT
   <213> Unknown
<220>
   <223> Obtained from genus Triticum species
<400> 42
   Asn Phe Leu Leu Gln Gln Cys Asn His Val Ser Leu Val Ser Ser Leu
   1               5                   10                  15
   Val Ser Ile Ile Leu Pro Arg
               20
<210> 43
   <211> 26
   <212> PRT
   <213> Unknown
<220>
   <223> Obtained from genus Triticum species
<400> 43
   Pro Ser Gly Gln Val Gln Trp Pro Gln Gln Gln Pro Phe Pro Gln Pro
   1               5                   10                  15
   Gln Gln Pro Phe Cys Gln Gln Pro Gln Arg
               20                  25
<210> 44
   <211> 7
   <212> PRT
   <213> Unknown
<220>
   <223> Obtained from genus Triticum species
<400> 44
   Val Asn Val Pro Leu Tyr Arg
   1 5
<210> 45
   <211> 10
   <212> PRT
   <213> Unknown
<220>
   <223> Obtained from genus Triticum species
<400> 45
   Leu Glu Val Met Thr Ser Ile Ala Leu Arg
   1 5 10
<210> 46
   <211> 14
   <212> PRT
   <213> Unknown
<220>
   <223> Obtained from genus Triticum species
<400> 46
   Thr Thr Thr Ser Val Pro Phe Gly Val Gly Thr Gly Val Gly
   1 5 10
<210> 47
   <211> 15
   <212> PRT
   <213> Unknown
<220>
   <223> Obtained from genus Triticum species
<400> 47
   Thr Thr Thr Ser Val Pro Phe Gly Val Gly Thr Gly Val Gly Ala
   1 5 10 15
<210> 48
   <211> 16
   <212> PRT
   <213> Unknown
<220>
   <223> Obtained from genus Triticum species
<400> 48
   Thr Thr Thr Ser Val Pro Phe Gly Val Gly Thr Gly Val Gly Ala Tyr
   1               5                   10                  15
<210> 49
   <211> 14
   <212> PRT
   <213> Unknown
<220>
   <223> Obtained from genus Triticum species
<400> 49
   Ile Leu Pro Thr Met Cys Ser Val Asn Val Pro Leu Tyr Arg
   1               5                   10
<210> 50
   <211> 28
   <212> PRT
   <213> Unknown
<220>
   <223> Obtained from genus Triticum species
<400> 50
   Ser Gln Met Leu Gln Gln Ser Ser Cys His Val Met Gln Gln Gln Cys
   1               5                   10                  15
   Cys Gln Gln Leu Pro Gln Ile Pro Gln Gln Ser Arg
               20                  25
<210> 51
   <211> 33
   <212> PRT
   <213> Unknown
<220>
   <223> Obtained from genus Triticum species
<400> 51
   Ser Gln Met Leu Gln Gln Ser Ser Cys His Val Met Gln Gln Gln Cys
   1               5                   10                  15
   Cys Gln Gln Leu Pro Gln Ile Pro Gln Gln Ser Arg Tyr Glu Ala Ile
               20                  25                  30
   Arg
<210> 52
   <211> 13
   <212> PRT
   <213> Unknown
<220>
   <223> Obtained from genus Triticum species
<400> 52
   Ala Pro Phe Ala Ser Ile Val Ala Gly Ile Gly Gly Gln
   1               5                   10
<210> 53
   <211> 13
   <212> PRT
   <213> Unknown
<220>
   <223> Obtained from genus Triticum species
<400> 53
   Ala Pro Phe Ala Ser Ile Val Ala Gly Ile Gly Gly Gln
   1               5                   10
<210> 54
   <211> 14
   <212> PRT
   <213> Unknown
<220>
   <223> Obtained from genus Triticum species
<400> 54
   Arg Ala Pro Phe Ala Ser Ile Val Ala Gly Ile Gly Gly Gln
   1               5                   10
<210> 55
   <211> 23
   <212> PRT
   <213> Unknown
<220>
   <223> Obtained from genus Triticum species
<400> 55
   Ser Leu Val Leu Gln Thr Leu Pro Ser Met Cys Asn Val Tyr Val Pro
   1               5                   10                  15
   Pro Glu Cys Ser Ile Met Arg
               20
<210> 56
   <211> 28
   <212> PRT
   <213> Unknown
<220>
   <223> Obtained from genus Triticum species
<400> 56
   Gln Gln Pro Phe Pro Gln Thr Gln Gln Pro Gln Gln Pro Phe Pro Gln
   1               5                   10                  15
   Gln Pro Gln Gln Pro Phe Pro Gln Thr Gln Gln Pro
               20                  25
<210> 57
   <211> 12
   <212> PRT
   <213> Unknown
<220>
   <223> Obtained from genus Triticum species
<400> 57
   Gln Ile Pro Glu Gln Ser Arg His Glu Ser Ile Arg
   1               5                   10
<210> 58
   <211> 14
   <212> PRT
   <213> Unknown
<220>
   <223> Obtained from genus Triticum species
<400> 58
   Val Phe Leu Gln Gln Gln Cys Ile Pro Val Ala Met Gln Arg
   1               5                   10
<210> 59
   <211> 19
   <212> PRT
   <213> Unknown
<220>
   <223> Obtained from genus Triticum species
<400> 59
   Gln Gln Gln Ile Pro Val Ile His Pro Ser Val Leu Gln Gln Leu Asn
   1               5                   10                  15
   Pro Cys Lys
<210> 60
   <211> 8
   <212> PRT
   <213> Unknown
<220>
   <223> Obtained from genus Triticum species
<400> 60
   Glu Leu Gln Glu Leu Gln Glu Arg
   1               5
<210> 61
   <211> 11
   <212> PRT
   <213> Unknown
<220>
   <223> Obtained from genus Triticum species
<400> 61
   Leu Glu Gly Gly Asp Ala Leu Ser Ala Ser Gln
   1               5                   10
<210> 62
   <211> 10
   <212> PRT
   <213> Unknown
<220>
   <223> Obtained from genus Triticum species
<400> 62
   Ile Phe Trp Gly Ile Pro Ala Leu Leu Lys
   1               5                   10
<210> 63
   <211> 10
   <212> PRT
   <213> Unknown
<220>
   <223> Obtained from genus Triticum species
<400> 63
   Ile Phe Trp Gly Ile Pro Ala Leu Leu Lys
   1               5                   10
<210> 64
   <211> 11
   <212> PRT
   <213> Unknown
<220>
   <223> Obtained from genus Triticum species
<400> 64
   Ile Phe Trp Gly Ile Pro Ala Leu Leu Lys Arg
   1 5 10
<210> 65
   <211> 11
   <212> PRT
   <213> Unknown
<220>
   <223> Obtained from genus Triticum species
<400> 65
   Gly Glu Ala Ser Glu Gln Leu Gln Cys Glu Arg
   1 5 10
<210> 66
   <211> 11
   <212> PRT
   <213> Unknown
<220>
   <223> Obtained from genus Triticum species
<400> 66
   Ile Phe Trp Gly Ile Pro Ala Leu Leu Lys Arg
   1 5 10
<210> 67
   <211> 12
   <212> PRT
   <213> Unknown
<220>
   <223> Obtained from genus Triticum species
<400> 67
   Glu Gly Glu Ala Ser Glu Gln Leu Gln Cys Glu Arg
   1 5 10
<210> 68
   <211> 13
   <212> PRT
   <213> Unknown
<220>
   <223> Obtained from genus Triticum species
<400> 68
   Ala Gln Gln Leu Ala Ala Gln Leu Pro Ala Met Cys Arg
   1 5 10
<210> 69
   <211> 9
   <212> PRT
   <213> Unknown
<220>
   <223> Obtained from genus Triticum species
<400> 69
   Ser Val Ala Val Ser Gln Val Ala Arg
   1 5
<210> 70
   <211> 10
   <212> PRT
   <213> Unknown
<220>
   <223> Obtained from genus Triticum species
<400> 70
   Gln Val Val Asp Gln Gln Leu Ala Gly Arg
   1 5 10
<210> 71
   <211> 11
   <212> PRT
   <213> Unknown
<220>
   <223> Obtained from genus Triticum species
<400> 71
   Glu Leu Gln Glu Ser Ser Leu Glu Ala Cys Arg
   1 5 10
<210> 72
   <211> 13
   <212> PRT
   <213> Unknown
<220>
   <223> Obtained from genus Triticum species
<400> 72
   Ala Gln Gln Pro Ala Thr Gln Leu Pro Thr Val Cys Arg
   1 5 10
<210> 73
   <211> 17
   <212> PRT
   <213> Unknown
<220>
   <223> Obtained from genus Triticum species
<400> 73
   Gln Leu Gln Cys Glu Arg Glu Leu Gln Glu Ser Ser Leu Glu Ala Cys
   1               5                   10                  15
   Arg
<210> 74
   <211> 20
   <212> PRT
   <213> Unknown
<220>
   <223> Obtained from genus Triticum species
<400> 74
   Gln Val Val Asp Gln Gln Leu Ala Gly Arg Leu Pro Trp Ser Thr Gly
   1               5                   10                  15
   Leu Gln Met Arg
               20
<210> 75
   <211> 37
   <212> PRT
   <213> Unknown
<220>
   <223> Obtained from genus Triticum species
<400> 75
   Gln Gln Pro Val Gln Gly Gln Gln Pro Glu Gln Gly Gln Gln Pro Gly
   1               5                   10                  15
   Gln Trp Gln Gln Gly Tyr Tyr Pro Thr Ser Pro Gln Gln Leu Gly Gln
               20                  25                  30
   Gly Gln Gln Pro Arg
           35
<210> 76
   <211> 35
   <212> PRT
   <213> Unknown
<220>
   <223> Obtained from genus Triticum species
<400> 76
   Gln Gly Tyr Tyr Pro Thr Ser Leu Gln Gln Pro Gly Gln Gly Gln Gln
   1               5                   10                  15
   Ile Gly Gln Gly Gln Gln Gly Tyr Tyr Pro Thr Ser Pro Gln His Thr
               20                  25                  30
   Gly Gln Arg
           35
<210> 77
   <211> 6
   <212> PRT
   <213> Unknown
<220>
   <223> Obtained from genus Triticum species
<400> 77
   Gln Trp Leu Gln Pro Arg
   1               5
<210> 78
   <211> 11
   <212> PRT
   <213> Unknown
<220>
   <223> Obtained from genus Triticum species
<400> 78
   Leu Glu Gly Gly Asp Ala Leu Leu Ala Ser Gln
   1               5                   10
<210> 79
   <211> 11
   <212> PRT
   <213> Unknown
<220>
   <223> Obtained from genus Triticum species
<400> 79
   Leu Glu Gly Gly Asp Ala Leu Leu Ala Ser Gln
   1               5                   10
<210> 80
   <211> 6
   <212> PRT
   <213> Unknown
<220>
   <223> Obtained from mix of genus Brassica species and genus Sinapis species
<400> 80
   Pro Ala Gly Pro Phe Arg
   1               5
<210> 81
   <211> 9
   <212> PRT
   <213> Unknown
<220>
   <223> Obtained from mix of genus Brassica species and genus Sinapis species
<400> 81
   Pro Ala Gly Pro Phe Arg Ile Pro Lys
   1               5
<210> 82
   <211> 10
   <212> PRT
   <213> Unknown
<220>
   <223> Obtained from mix of genus Brassica species and genus Sinapis species
<400> 82
   Ile Tyr Gln Thr Ala Thr His Leu Pro Lys
   1               5                   10
<210> 83
   <211> 8
   <212> PRT
   <213> Unknown
<220>
   <223> Obtained from mix of genus Brassica species and genus Sinapis species
<400> 83
   Gln Val Ser Val Cys Pro Phe Lys
   1               5
<210> 84
   <211> 9
   <212> PRT
   <213> Unknown
<220>
   <223> Obtained from mix of genus Brassica species and genus Sinapis species
<400> 84
   Gln Val Ser Val Cys Pro Phe Lys Lys
   1 5
<210> 85
   <211> 9
   <212> PRT
   <213> Unknown
<220>
   <223> Obtained from mix of genus Brassica species and genus Sinapis species
<400> 85
   Gln Val Ser Val Cys Pro Phe Lys Lys
   1 5
<210> 86
   <211> 9
   <212> PRT
   <213> Unknown
<220>
   <223> Obtained from mix of genus Brassica species and genus Sinapis species
<400> 86
   Pro Ala Gly Pro Phe Gly Ile Pro Lys
   1 5
<210> 87
   <211> 19
   <212> PRT
   <213> Unknown
<220>
   <223> Obtained from mix of genus Brassica species and genus Sinapis species
<400> 87
   Gln Gln Leu Glu Gln Gln Gly Gln Gln Gly Pro His Leu Gln His Val
   1 5 10 15
   Ile Ser Arg
<210> 88
   <211> 19
   <212> PRT
   <213> Unknown
<220>
   <223> Obtained from mix of genus Brassica species and genus Sinapis species
<400> 88
   Gln Gln Leu Glu Gln Gln Gly Gln Gln Gly Pro His Leu Gln His Val
   1 5 10 15
   Ile Ser Arg
<210> 89
   <211> 10
   <212> PRT
   <213> Unknown
<220>
   <223> Obtained from mix of genus Brassica species and genus Sinapis species
<400> 89
   Ile Tyr Gln Thr Ala Thr His Leu Pro Arg
   1 5 10
<210> 90
   <211> 10
   <212> PRT
   <213> Unknown
<220>
   <223> Obtained from mix of genus Brassica species and genus Sinapis species
<400> 90
   Ile Tyr Gln Thr Ala Thr His Leu Pro Arg
   1 5 10
<210> 91
   <211> 9
   <212> PRT
   <213> Unknown
<220>
   <223> Obtained from mix of genus Brassica species and genus Sinapis species
<400> 91
   Gln Val Ser Val Cys Pro Phe Gln Lys
   1 5
<210> 92
   <211> 9
   <212> PRT
   <213> Unknown
<220>
   <223> Obtained from mix of genus Brassica species and genus Sinapis species
<400> 92
   Gln Val Ser Val Cys Pro Phe Gln Lys
   1 5
<210> 93
   <211> 13
   <212> PRT
   <213> Unknown
<220>
   <223> Obtained from mix of genus Brassica species and genus Sinapis species
<400> 93
   Val Cys Asn Ile Pro Gln Val Ser Val Cys Pro Phe Lys
   1               5                   10
<210> 94
   <211> 13
   <212> PRT
   <213> Unknown
<220>
   <223> Obtained from mix of genus Brassica species and genus Sinapis species
<400> 94
   Val Cys Asn Ile Pro Gln Val Ser Val Cys Pro Phe Lys
   1               5                   10
<210> 95
   <211> 14
   <212> PRT
   <213> Unknown
<220>
   <223> Obtained from mix of genus Brassica species and genus Sinapis species
<400> 95
   Val Cys Asn Ile Pro Gln Val Ser Val Cys Pro Phe Lys Lys
   1               5                   10
<210> 96
   <211> 14
   <212> PRT
   <213> Unknown
<220>
   <223> Obtained from mix of genus Brassica species and genus Sinapis species
<400> 96
   Val Cys Asn Ile Pro Gln Val Ser Val Cys Pro Phe Lys Lys
   1               5                   10
<210> 97
   <211> 19
   <212> PRT
   <213> Unknown
<220>
   <223> Obtained from mix of genus Brassica species and genus Sinapis species
<400> 97
   Gln Gln Leu Gly Gln Gln Gly Gln Gln Gly Pro Gln Val Gln His Val
   1               5                   10                  15
   Ile Ser Arg
<210> 98
   <211> 19
   <212> PRT
   <213> Unknown
<220>
   <223> Obtained from mix of genus Brassica species and genus Sinapis species
<400> 98
   Gln Gln Leu Gly Gln Gln Gly Gln Gln Gly Pro Gln Val Gln His Val
   1               5                   10                  15
   Ile Ser Arg
<210> 99
   <211> 10
   <212> PRT
   <213> Unknown
<220>
   <223> Obtained from mix of genus Brassica species and genus Sinapis species
<400> 99
   Ile Tyr Gln Thr Ala Thr His Leu Pro Arg
   1               5                   10
<210> 100
   <211> 5
   <212> PRT
   <213> Unknown
<220>
   <223> Obtained from mix of genus Brassica species and genus Sinapis species
<400> 100
   Ala Gly Pro Phe Arg
   1               5
<210> 101
   <211> 7
   <212> PRT
   <213> Unknown
<220>
   <223> Obtained from mix of genus Brassica species and genus Sinapis species
<400> 101
   Ala Val Lys Gln Gln Ile Arg
   1 5
<210> 102
   <211> 19
   <212> PRT
   <213> Unknown
<220>
   <223> Obtained from mix of genus Brassica species and genus Sinapis species
<400> 102
   Gln Gln Gly Gln Gln Gln Gly Gln Gln Gly Gln Gln Leu Gln His Glu
   1 5 10 15
   Ile Ser Arg
<210> 103
   <211> 6
   <212> PRT
   <213> Unknown
<220>
   <223> Obtained from mix of genus Brassica species and genus Sinapis species
<400> 103
   Val Cys Asn Ile Pro Arg
   1 5
<210> 104
   <211> 8
   <212> PRT
   <213> Unknown
<220>
   <223> Obtained from mix of genus Brassica species and genus Sinapis species
<400> 104
   Val Ser Ile Cys Pro Phe Gln Lys
   1 5
<210> 105
   <211> 8
   <212> PRT
   <213> Unknown
<220>
   <223> Obtained from mix of genus Brassica species and genus Sinapis species
<400> 105
   Val Ser Ile Cys Pro Phe Gln Lys
   1 5
<210> 106
   <211> 9
   <212> PRT
   <213> Unknown
<220>
   <223> Obtained from mix of genus Brassica species and genus Sinapis species
<400> 106
   Glu Phe Arg Gln Ala Gln His Leu Arg
   1 5
<210> 107
   <211> 9
   <212> PRT
   <213> Unknown
<220>
   <223> Obtained from mix of genus Brassica species and genus Sinapis species
<400> 107
   Glu Phe Arg Gln Ala Gln His Leu Arg
   1 5
<210> 108
   <211> 6
   <212> PRT
   <213> Unknown
<220>
   <223> Obtained from mix of genus Brassica species and genus Sinapis species
<400> 108
   Gln Ala Gln His Leu Arg
   1 5
<210> 109
   <211> 8
   <212> PRT
   <213> Unknown
<220>
   <223> Obtained from mix of genus Brassica species and genus Sinapis species
<400> 109
   Ala Cys Gln Gln Trp Leu His Arg
   1 5
<210> 110
   <211> 18
   <212> PRT
   <213> Unknown
<220>
   <223> Obtained from mix of genus Brassica species and genus Sinapis species
<400> 110
   Gln Gln Val Arg Gln Gln Gly His Gln Gln Gln Met Gln His Val Ile
   1               5                   10                  15
   Ser Arg
<210> 111
   <211> 18
   <212> PRT
   <213> Unknown
<220>
   <223> Obtained from mix of genus Brassica species and genus Sinapis species
<400> 111
   Gln Gln Val Arg Gln Gln Gly His Gln Gln Gln Met Gln His Val Ile
   1               5                   10                  15
   Ser Arg
<210> 112
   <211> 10
   <212> PRT
   <213> Unknown
<220>
   <223> Obtained from mix of genus Brassica species and genus Sinapis species
<400> 112
   Ile Tyr Gln Thr Ala Thr His Leu Pro Arg
   1               5                   10
<210> 113
   <211> 17
   <212> PRT
   <213> Unknown
<220>
   <223> Obtained from Avena sativa
<400> 113
   Gln Phe Leu Val Gln Gln Cys Ser Pro Val Ala Val Val Pro Phe Leu
   1               5                   10                  15
   Arg
<210> 114
   <211> 13
   <212> PRT
   <213> Unknown
<220>
   <223> Obtained from Avena sativa
<400> 114
   Ser Gln Ile Leu Gln Gln Ser Ser Cys Gln Val Met Arg
   1               5                   10
<210> 115
   <211> 10
   <212> PRT
   <213> Unknown
<220>
   <223> Obtained from Avena sativa
<400> 115
   Gln Leu Glu Gln Ile Pro Glu Gln Leu Arg
   1               5                   10
<210> 116
   <211> 10
   <212> PRT
   <213> Unknown
<220>
   <223> Obtained from Avena sativa
<400> 116
   Gln Leu Glu Gln Ile Pro Glu Gln Leu Arg
   1               5                   10
<210> 117
   <211> 17
   <212> PRT
   <213> Unknown
<220>
   <223> Obtained from genus Secalin
<400> 117
   Asn Val Leu Leu Gln Gln Cys Ser Pro Val Ala Leu Val Ser Ser Leu
   1               5                   10                  15
   Arg
<210> 118
   <211> 17
   <212> PRT
   <213> Unknown
<220>
   <223> Obtained from genus Secalin
<400> 118
   Asn Val Leu Leu Gln Gln Cys Ser Pro Val Ala Leu Val Ser Ser Leu
   1               5                   10                  15
   Arg
<210> 119
   <211> 39
   <212> PRT
   <213> Unknown
<220>
   <223> Obtained from genus Secalin
<400> 119
   Glu Gly Val Gln Ile Leu Leu Pro Gln Ser His Gln Gln His Val Gly
   1               5                   10                  15
   Gln Gly Ala Leu Ala Gln Val Gln Gly Ile Ile Gln Pro Gln Gln Leu
               20                  25                  30
   Ser Gln Leu Glu Val Val Arg
           35
<210> 120
   <211> 17
   <212> PRT
   <213> Unknown
<220>
   <223> Obtained from genus Secalin
<400> 120
   Ser Leu Val Leu Gln Asn Leu Pro Thr Met Cys Asn Val Tyr Val Pro
   1               5                   10                  15
   Arg
<210> 121
   <211> 17
   <212> PRT
   <213> Unknown
<220>
   <223> Obtained from genus Secalin
<400> 121
   Ser Leu Val Leu Gln Asn Leu Pro Thr Met Cys Asn Val Tyr Val Pro
   1               5                   10                  15
   Arg
<210> 122
   <211> 19
   <212> PRT
   <213> Unknown
<220>
   <223> Obtained from genus Secalin
<400> 122
   Gln Cys Ser Thr Ile Gln Ala Pro Phe Ala Ser Ile Val Thr Gly Ile
   1               5                   10                  15
   Val Gly His
<210> 123
   <211> 19
   <212> PRT
   <213> Unknown
<220>
   <223> Obtained from genus Secalin
<400> 123
   Gln Cys Ser Thr Ile Gln Ala Pro Phe Ala Ser Ile Val Thr Gly Ile
   1               5                   10                  15
   Val Gly His
<210> 124
   <211> 9
   <212> PRT
   <213> Unknown
<220>
   <223> Obtained from genus Sesamum
<400> 124
   Ile Ser Gly Ala Gln Pro Ser Leu Arg
   1               5
<210> 125
   <211> 6
   <212> PRT
   <213> Unknown
<220>
   <223> Obtained from genus Sesamum
<400> 125
   Leu Val Tyr Ile Glu Arg
   1 5
<210> 126
   <211> 19
   <212> PRT
   <213> Unknown
<220>
   <223> Obtained from genus Sesamum
<400> 126
   Ala Phe Asp Ala Glu Leu Leu Ser Glu Ala Phe Asn Val Pro Gln Glu
   1 5 10 15
   Thr Ile Arg
<210> 127
   <211> 7
   <212> PRT
   <213> Unknown
<220>
   <223> Obtained from genus Sesamum
<400> 127
   Gly Leu Ile Val Met Ala Arg
   1 5
<210> 128
   <211> 7
   <212> PRT
   <213> Unknown
<220>
   <223> Obtained from genus Sesamum
<400> 128
   Glu Ala Asp Ile Phe Ser Arg
   1 5
<210> 129
   <211> 11
   <212> PRT
   <213> Unknown
<220>
   <223> Obtained from genus Sesamum
<400> 129
   Ser Pro Leu Ala Gly Tyr Thr Ser Val Ile Arg
   1 5 10
<210> 130
   <211> 22
   <212> PRT
   <213> Unknown
<220>
   <223> Obtained from genus Sesamum
<400> 130
   Ala Met Pro Leu Gln Val Ile Thr Asn Ser Tyr Gln Ile Ser Pro Asn
   1 5 10 15
   Gln Ala Gln Ala Leu Lys
   20
<210> 131
   <211> 7
   <212> PRT
   <213> Unknown
<220>
   <223> Obtained from genus Sesamum
<400> 131
   His Cys Met Gln Trp Met Arg
   1 5
<210> 132
   <211> 11
   <212> PRT
   <213> Unknown
<220>
   <223> Obtained from genus Sesamum
<400> 132
   Met Cys Gly Met Ser Tyr Pro Thr Glu Cys Arg
   1 5 10
<210> 133
   <211> 9
   <212> PRT
   <213> Unknown
<220>
   <223> Obtained from genus Triticum
<400> 133
   Ser Val Ala Val Ser Gln Val Ala Arg
   1 5
<210> 134
   <211> 16
   <212> PRT
   <213> Unknown
<220>
   <223> Obtained from genus Triticum
<400> 134
   Glu His Gly Ala Gln Glu Gly Gln Ala Gly Thr Gly Ala Phe Pro Arg
   1 5 10 15
<210> 135
   <211> 10
   <212> PRT
   <213> Unknown
<220>
   <223> Obtained from genus Triticum
<400> 135
   Gln Val Val Asp Gln Gln Leu Ala Gly Arg
   1 5 10
<210> 136
   <211> 10
   <212> PRT
   <213> Unknown
<220>
   <223> Obtained from genus Triticum
<400> 136
   Ile Phe Trp Gly Ile Pro Ala Leu Leu Lys
   1 5 10
<210> 137
   <211> 10
   <212> PRT
   <213> Unknown
<220>
   <223> Obtained from genus Triticum
<400> 137
   Leu Pro Trp Ser Thr Gly Leu Gln Met Arg
   1 5 10
<210> 138
   <211> 11
   <212> PRT
   <213> Unknown
<220>
   <223> Obtained from genus Triticum
<400> 138
   Tyr Asp Pro Thr Ala Tyr Asn Thr Ile Leu Arg
   1               5                   10
<210> 139
   <211> 8
   <212> PRT
   <213> Unknown
<220>
   <223> Obtained from Bertolletia excelsa
<400> 139
   Gln Gln Met Leu Ser His Cys Arg
   1               5
<210> 140
   <211> 21
   <212> PRT
   <213> Unknown
<220>
   <223> Obtained from Bertolletia excelsa
<400> 140
   Gly Met Glu Pro His Met Ser Glu Cys Cys Glu Gln Leu Glu Gly Met
   1               5                   10                  15
   Asp Glu Ser Cys Arg
               20
<210> 141
   <211> 5
   <212> PRT
   <213> Unknown
<220>
   <223> Obtained from Bertolletia excelsa
<400> 141
   Met Met Met Met Arg
   1               5
<210> 142
   <211> 9
   <212> PRT
   <213> Unknown
<220>
   <223> Obtained from Bertolletia excelsa
<400> 142
   Met Gln Gln Glu Glu Met Gln Pro Arg
   1               5
<210> 143
   <211> 8
   <212> PRT
   <213> Unknown
<220>
   <223> Obtained from Bertolletia excelsa
<400> 143
   Leu Ala Glu Asn Ile Pro Ser Arg
   1               5
<210> 144
   <211> 7
   <212> PRT
   <213> Unknown
<220>
   <223> Obtained from Bertolletia excelsa
<400> 144
   Cys Asn Leu Ser Pro Met Arg
   1               5
<210> 145
   <211> 8
   <212> PRT
   <213> Unknown
<220>
   <223> Obtained from Bertolletia excelsa
<400> 145
   Gln Gln Gln Leu Asn His Cys Arg
   1               5
<210> 146
   <211> 6
   <212> PRT
   <213> Unknown
<220>
   <223> Obtained from Bertolletia excelsa
<400> 146
   Met Asp Glu Met Cys Arg
   1 5
<210> 147
   <211> 7
   <212> PRT
   <213> Unknown
<220>
   <223> Obtained from Bertolletia excelsa
<400> 147
   Cys Asn Leu Ser Pro Gln Arg
   1 5
<210> 148
   <211> 8
   <212> PRT
   <213> Unknown
<220>
   <223> Obtained from Bertolletia excelsa
<400> 148
   Cys Ala Gly Val Ala Ala Leu Arg
   1 5
<210> 149
   <211> 8
   <212> PRT
   <213> Unknown
<220>
   <223> Obtained from Bertolletia excelsa
<400> 149
   Leu Tyr Tyr Val Thr Gln Gly Arg
   1 5
<210> 150
   <211> 10
   <212> PRT
   <213> Unknown
<220>
   <223> Obtained from Bertolletia excelsa
<400> 150
   His Phe Phe Leu Ala Gly Asn Ile Gln Arg
   1 5 10
<210> 151
   <211> 12
   <212> PRT
   <213> Unknown
<220>
   <223> Obtained from Bertolletia excelsa
<400> 151
   Gly Gly Gln Gln Ile Leu Ala Asp Asn Val Phe Lys
   1               5                   10
<210> 152
   <211> 21
   <212> PRT
   <213> Unknown
<220>
   <223> Obtained from Bertolletia excelsa
<400> 152
   Gly Phe Asn Met Glu Ala Leu Ala Asp Val Leu Gly Phe Gly Met Asp
   1               5                   10                  15
   Thr Glu Thr Ala Arg
               20
<210> 153
   <211> 14
   <212> PRT
   <213> Unknown
<220>
   <223> Obtained from Bertolletia excelsa
<400> 153
   Gly Val Leu Tyr Glu Asn Ala Met Met Ala Pro Leu Trp Arg
   1               5                   10
<210> 154
   <211> 12
   <212> PRT
   <213> Unknown
<220>
   <223> Obtained from Bertolletia excelsa
<400> 154
   Gly Ile Pro Val Gly Val Leu Ala Asn Ala Tyr Arg
   1               5                   10
<210> 155
   <211> 11
   <212> PRT
   <213> Unknown
<220>
   <223> Obtained from Bertolletia excelsa
<400> 155
   Asp Glu Ala Val Leu Phe Gln Pro Gly Ser Arg
   1 5 10
<210> 156
   <211> 9
   <212> PRT
   <213> Unknown
<220>
   <223> Obtained from Corylus avellana
<400> 156
   Tyr Phe Gly Glu Cys Asn Leu Asp Arg
   1 5
<210> 157
   <211> 9
   <212> PRT
   <213> Unknown
<220>
   <223> Obtained from genus Corylus
<400> 157
   Leu Asn Ala Leu Glu Pro Thr Asn Arg
   1 5
<210> 158
   <211> 23
   <212> PRT
   <213> Unknown
<220>
   <223> Obtained from genus Corylus
<400> 158
   Thr Ile Glu Pro Asn Gly Leu Leu Leu Pro Gln Tyr Ser Asn Ala Pro
   1 5 10 15
   Glu Leu Ile Tyr Ile Glu Arg
   20
<210> 159
   <211> 14
   <212> PRT
   <213> Unknown
<220>
   <223> Obtained from genus Corylus
<400> 159
   His Phe Tyr Leu Ala Gly Asn Pro Asp Asp Glu His Gln Arg
   1               5                   10
<210> 160
   <211> 9
   <212> PRT
   <213> Unknown
<220>
   <223> Obtained from genus Corylus
<400> 160
   Gln Gly Gln Gln Gln Phe Gly Gln Arg
   1               5
<210> 161
   <211> 5
   <212> PRT
   <213> Unknown
<220>
   <223> Obtained from genus Corylus
<400> 161
   Gln Glu Trp Glu Arg
   1               5
<210> 162
   <211> 10
   <212> PRT
   <213> Unknown
<220>
   <223> Obtained from genus Corylus
<400> 162
   Ala Asp Ile Tyr Thr Glu Gln Val Gly Arg
   1               5                   10
<210> 163
   <211> 13
   <212> PRT
   <213> Unknown
<220>
   <223> Obtained from genus Corylus
<400> 163
   Ile Asn Thr Val Asn Ser Asn Thr Leu Pro Val Leu Arg
   1               5                   10
<210> 164
   <211> 8
   <212> PRT
   <213> Unknown
<220>
   <223> Obtained from genus Corylus
<400> 164
   Trp Leu Gln Leu Ser Ala Glu Arg
   1               5
<210> 165
   <211> 15
   <212> PRT
   <213> Unknown
<220>
   <223> Obtained from genus Corylus
<400> 165
   Gln Gly Gln Val Leu Thr Ile Pro Gln Asn Phe Ala Val Ala Lys
   1               5                   10                  15
<210> 166
   <211> 12
   <212> PRT
   <213> Unknown
<220>
   <223> Obtained from genus Corylus
<400> 166
   Ala Glu Ser Glu Gly Phe Glu Trp Val Ala Phe Lys
   1               5                   10
<210> 167
   <211> 13
   <212> PRT
   <213> Unknown
<220>
   <223> Obtained from genus Corylus
<400> 167
   Thr Asn Asp Asn Ala Gln Ile Ser Pro Leu Ala Gly Arg
   1               5                   10
<210> 168
   <211> 15
   <212> PRT
   <213> Unknown
<220>
   <223> Obtained from genus Corylus
<400> 168
   Ala Leu Pro Asp Asp Val Leu Ala Asn Ala Phe Gln Ile Ser Arg
   1               5                   10                  15
<210> 169
   <211> 7
   <212> PRT
   <213> Unknown
<220>
   <223> Obtained from genus Corylus
<400> 169
   Gln Glu Thr Thr Leu Val Arg
   1               5
<210> 170
   <211> 10
   <212> PRT
   <213> Unknown
<220>
   <223> Obtained from genus Corylus
<400> 170
   Val Glu Glu Ile Asp His Ala Asn Phe Lys
   1               5                   10
<210> 171
   <211> 14
   <212> PRT
   <213> Unknown
<220>
   <223> Obtained from genus Corylus
<400> 171
   Ala Val Glu Ala Tyr Leu Leu Ala His Pro Asp Ala Tyr Cys
   1               5                   10
<210> 172
   <211> 14
   <212> PRT
   <213> Unknown
<220>
   <223> Obtained from genus Corylus
<400> 172
   Ala Val Glu Ala Tyr Leu Leu Ala His Pro Asp Ala Tyr Cys
   1               5                   10
<210> 173
   <211> 12
   <212> PRT
   <213> Unknown
<220>
   <223> Obtained from genus Macadamia
<400> 173
   Gln Cys Met Gln Leu Glu Thr Ser Gly Gln Met Arg
   1               5                   10
<210> 174
   <211> 7
   <212> PRT
   <213> Unknown
<220>
   <223> Obtained from genus Macadamia
<400> 174
   Cys Val Ser Gln Cys Asp Lys
   1               5
<210> 175
   <211> 9
   <212> PRT
   <213> Unknown
<220>
   <223> Obtained from genus Macadamia
<400> 175
   Phe Glu Glu Asp Ile Asp Trp Ser Lys
   1               5
<210> 176
   <211> 8
   <212> PRT
   <213> Unknown
<220>
   <223> Obtained from genus Macadamia
<400> 176
   His Met Gln Ile Cys Gln Gln Arg
   1               5
<210> 177
   <211> 8
   <212> PRT
   <213> Unknown
<220>
   <223> Obtained from genus Macadamia
<400> 177
   His Cys Glu Gln Gln Glu Pro Arg
   1 5
<210> 178
   <211> 7
   <212> PRT
   <213> Unknown
<220>
   <223> Obtained from genus Macadamia
<400> 178
   Leu Gln Tyr Gln Cys Gln Arg
   1 5
<210> 179
   <211> 6
   <212> PRT
   <213> Unknown
<220>
   <223> Obtained from genus Macadamia
<400> 179
   Glu Gly Val Ile Ile Arg
   1 5
<210> 180
   <211> 6
   <212> PRT
   <213> Unknown
<220>
   <223> Obtained from genus Macadamia
<400> 180
   Glu Ser Glu Phe Asp Arg
   1 5
<210> 181
   <211> 7
   <212> PRT
   <213> Unknown
<220>
   <223> Obtained from genus Macadamia
<400> 181
   Gln Gln Tyr Gln Cys Gln Arg
   1 5
<210> 182
   <211> 9
   <212> PRT
   <213> Unknown
<220>
   <223> Obtained from Arachis hypogaea
<400> 182
   Asn Leu Pro Gln Gln Cys Gly Leu Arg
   1 5
<210> 183
   <211> 9
   <212> PRT
   <213> Unknown
<220>
   <223> Obtained from Arachis hypogaea
<400> 183
   Asn Asn Pro Phe Tyr Phe Pro Ser Arg
   1 5
<210> 184
   <211> 11
   <212> PRT
   <213> Unknown
<220>
   <223> Obtained from Arachis hypogaea
<400> 184
   Thr Ala Asn Asp Leu Asn Leu Leu Ile Leu Arg
   1 5 10
<210> 185
   <211> 13
   <212> PRT
   <213> Unknown
<220>
   <223> Obtained from Arachis hypogaea
<400> 185
   Asp Leu Ala Phe Pro Gly Ser Gly Glu Gln Val Glu Lys
   1 5 10
<210> 186
   <211> 13
   <212> PRT
   <213> Unknown
<220>
   <223> Obtained from Arachis hypogaea
<400> 186
   Ser Pro Asp Ile Tyr Asn Pro Gln Ala Gly Ser Leu Lys
   1               5                   10
<210> 187
   <211> 14
   <212> PRT
   <213> Unknown
<220>
   <223> Obtained from Arachis hypogaea
<400> 187
   Val Leu Leu Glu Glu Asn Ala Gly Gly Glu Gln Glu Glu Arg
   1               5                   10
<210> 188
   <211> 8
   <212> PRT
   <213> Unknown
<220>
   <223> Obtained from Pistacia vera
<400> 188
   Cys Ala Gly Val Ala Val Ala Arg
   1               5
<210> 189
   <211> 9
   <212> PRT
   <213> Unknown
<220>
   <223> Obtained from Pistacia vera
<400> 189
   His Thr Ile Gln Pro Asn Gly Leu Arg
   1               5
<210> 190
   <211> 14
   <212> PRT
   <213> Unknown
<220>
   <223> Obtained from Pistacia vera
<400> 190
   Ile Leu Ala Glu Val Phe Gln Val Glu Gln Ser Leu Val Lys
   1 5 10
<210> 191
   <211> 12
   <212> PRT
   <213> Unknown
<220>
   <223> Obtained from Pistacia vera
<400> 191
   Gly Phe Glu Ser Glu Glu Glu Ser Glu Tyr Glu Arg
   1 5 10
<210> 192
   <211> 10
   <212> PRT
   <213> Unknown
<220>
   <223> Obtained from Pistacia vera
<400> 192
   Ser Asp Ile Tyr Thr Pro Glu Val Gly Arg
   1 5 10
<210> 193
   <211> 13
   <212> PRT
   <213> Unknown
<220>
   <223> Obtained from Pistacia vera
<400> 193
   Ile Thr Ser Leu Asn Ser Leu Asn Leu Pro Ile Leu Lys
   1 5 10
<210> 194
   <211> 8
   <212> PRT
   <213> Unknown
<220>
   <223> Obtained from Pistacia vera
<400> 194
   Trp Leu Gln Leu Ser Ala Glu Arg
   1 5
<210> 195
   <211> 7
   <212> PRT
   <213> Unknown
<220>
   <223> Obtained from Pistacia veram
<400> 195
   Phe Glu Trp Ile Ser Phe Lys
   1               5
<210> 196
   <211> 14
   <212> PRT
   <213> Unknown
<220>
   <223> Obtained from Pistacia vera
<400> 196
   Ala Met Ile Ser Pro Leu Ala Gly Ser Thr Ser Val Leu Arg
   1               5                   10
<210> 197
   <211> 15
   <212> PRT
   <213> Unknown
<220>
   <223> Obtained from Pistacia vera
<400> 197
   Ala Met Pro Glu Glu Val Leu Ala Asn Ala Phe Gln Ile Ser Arg
   1               5                   10                  15
<210> 198
   <211> 16
   <212> PRT
   <213> Unknown
<220>
   <223> Obtained from Pistacia vera
<400> 198
   Phe Gln Thr Gln Cys Gln Ile Gln Asn Leu Asn Ala Leu Glu Pro Lys
   1               5                   10                  15
<210> 199
   <211> 27
   <212> PRT
   <213> Unknown
<220>
   <223> Obtained from Pistacia vera
<400> 199
   Ile Glu Ser Glu Ala Gly Val Thr Glu Phe Trp Asp Gln Asn Glu Glu
   1               5                   10                  15
   Gln Leu Gln Cys Ala Asn Val Ala Val Phe Arg
               20                  25
<210> 200
   <211> 21
   <212> PRT
   <213> Unknown
<220>
   <223> Obtained from Pistacia vera
<400> 200
   Leu Val Leu Val Ala Leu Ala Asp Val Gly Asn Ser Glu Asn Gln Leu
   1               5                   10                  15
   Asp Gln Tyr Leu Arg
               20
<210> 201
   <211> 5
   <212> PRT
   <213> Unknown
<220>
   <223> Obtained from Pistacia vera
<400> 201
   Gly Ile Ile Val Arg
   1               5
<210> 202
   <211> 15
   <212> PRT
   <213> Unknown
<220>
   <223> Obtained from Pistacia vera
<400> 202
   Gly Leu Pro Leu Asp Val Ile Gln Asn Ser Phe Asp Ile Ser Arg
   1               5                   10                  15
<210> 203
   <211> 11
   <212> PRT
   <213> Unknown
<220>
   <223> Obtained from Pistacia vera
<400> 203
   Ser Glu Met Thr Ile Phe Ala Pro Gly Ser Arg
   1 5 10
<210> 204
   <211> 12
   <212> PRT
   <213> Unknown
<220>
   <223> Obtained from Juglans regia
<400> 204
   Gln Gln Asn Leu Asn His Cys Gln Tyr Tyr Leu Arg
   1 5 10
<210> 205
   <211> 19
   <212> PRT
   <213> Unknown
<220>
   <223> Obtained from Juglans regia
<400> 205
   Gln Cys Cys Gln Gln Leu Ser Gln Met Asp Glu Gln Cys Gln Cys Glu
   1 5 10 15
   Gly Leu Arg
<210> 206
   <211> 12
   <212> PRT
   <213> Unknown
<220>
   <223> Obtained from Juglans regia
<400> 206
   Gly Glu Glu Met Glu Glu Met Val Gln Ser Ala Arg
   1 5 10
<210> 207
   <211> 12
   <212> PRT
   <213> Unknown
<220>
   <223> Obtained from Juglans regia
<400> 207
   Asp Leu Pro Asn Glu Cys Gly Ile Ser Ser Gln Arg
   1               5                   10
<210> 208
   <211> 9
   <212> PRT
   <213> Unknown
<220>
   <223> Obtained from Juglans regia
<400> 208
   Leu Asp Ala Leu Glu Pro Thr Asn Arg
   1               5
<210> 209
   <211> 27
   <212> PRT
   <213> Unknown
<220>
   <223> Obtained from Juglans regia
<400> 209
   Gly Leu Gly Asn Asn Val Phe Ser Gly Phe Asp Ala Asp Phe Leu Ala
   1               5                   10                  15
   Asp Ala Phe Asn Val Asp Thr Glu Thr Ala Arg
               20                  25
<210> 210
   <211> 8
   <212> PRT
   <213> Unknown
<220>
   <223> Obtained from Juglans regia
<400> 210
   Trp Leu Gln Leu Ser Ala Glu Arg
   1               5
<210> 211
   <211> 15
   <212> PRT
   <213> Unknown
<220>
   <223> Obtained from Juglans regia
<400> 211
   Ala Leu Pro Glu Glu Val Leu Ala Thr Ala Phe Gln Ile Pro Arg
   1               5                   10                  15
<210> 212
   <211> 15
   <212> PRT
   <213> Unknown
<220>
   <223> Obtained from Juglans regia
<400> 212
   Ala Leu Pro Glu Glu Val Leu Ala Thr Ala Phe Gln Ile Pro Arg
   1               5                   10                  15